# EUROPEAN PATENT APPLICATION

(11) **EP 2 260 844 A1**
(43) Date of publication of application: **15.12.2010**
(21) Application number: 10175680.7
(22) Date of filing: 27.05.2004
(51) Int. Cl.: A61K 31/343, A61K 45/06, A61P 25/24

(54) **Combination of an NMDA Receptor Antagonist and a Selective Serotonin Reuptake Inhibitor for the Treatment of Depression and other Mood Disorders**

(30) Priority: 27.05.2003 US 473639 P
(62) Divisional of application: 04776170.5
(71) Applicant: Merz Pharma GmbH & Co. KGaA, 60318 Frankfurt (DE)
(72) Inventor: Gupta, Sandeep, Plainsboro, New Jersey 08536 (US); Samoriski, Gary, Hilsborough, New Jersey 08844 (US)
(74) Representative: Goddard, Christopher Robert

(57) **Abstract**

The present invention provides a method for the treatment of depression, including treatment-resistant depression, and other mood disorders using a combination of an NMDA receptor antagonist and a SSRI, a NARI, a dual SSRI/NARI (a SNRI), or a tricyclic antidepressant (TCA). It has unexpectedly been shown that the combination has a synergistic and potentiated effect of either compound as monotherapy, resulting in an enhanced therapeutic effect at lower doses.

## Description

This application claims priority from U.S. provisional application Serial No. 60/473,639, filed May 27, 2003, which is herein incorporated by reference in its entirety.

### FIELD OF THE INVENTION

The present invention relates to a method for the treatment of depression, including treatment-resistant depression, and other mood disorders using a combination of an NMDA receptor antagonist and citalopram or escitalopram.

### BACKGROUND OF THE INVENTION

### Mood Disorders

Mood disorders such as depression, bipolar disorder, and seasonal affective disorder are typically treated with several classes of anti-depressants. The classical psychopharmaceuticals effective in the treatment of mood disorders can be grouped into three classes: the tricyclic antidepressants (TCAs), monoamine oxidase inhibitors (MAOIs) and lithium salts. While TCA and MAOI drugs are indicated for the depressive phase of the bipolar disorder, lithium is known to attenuate the bipolar mood swings. Newer antidepressants include selective serotonin reuptake inhibitors (SSRIs), selective norepinephrine reuptake inhibitors (NARIs), and dual SSRI/NARIs, called SNRIs.

The TCAs block the reuptake of monoamines, thereby elevating levels of noradrenaline and serotonin (5-hydroxytryptamine; 5-HT). Imipramine and amitriptyline are examples of TCAs. The SSRIs regulate serotonin. Trazodone controls the actions of 5-HT while fluoxetine is a potent and selective inhibitor of 5-HT reuptake. 3- chloroimipramine, which inhibits both 5-HT and norepinephrine reuptake has been extensively used as an antidepressant in Europe and Canada. Other compounds which are of current interest or have been examined as antidepressants include fluvoxamine (Luvox®), citalopram (Celexa®), escitalopram (Lexapro®), sertraline (Zoloft®), bupropion (Wellbutrin®, Zyban®), reboxetine (Edronax®), and mirtazapine (Remeron®). Fluvoxamine facilitates serotoninergic neurotransmission via potent and selective inhibition of serotonin reuptake into presynaptic neurons. Reboxetine is a selective norepinephrine reuptake inhibitor with potential utility in the treatment of severe depression. Still other compounds such as milnacipran (Ixel®), venlafaxine (Effexor®), duloxetine (Cymbalta®), mirtazapine and atomoxetine (Strattera®) block both 5-HT and norepinephrine reuptake.

Many of these compounds, especially TCAs, have adverse side effects when administered at therapeutic levels. The adverse effects occurring most frequently during treatment with SSRIs are gastrointestinal disturbances such as nausea, diarrhea/loose stools, constipation, with an incidence of 6 to 37% (Leonard et al., Drugs 1992; 43(Suppl. 2):3-9), and sexual dysfunction. Nausea is the main adverse effect in terms of incidence. These adverse effects, although mild to moderate in severity, deter some patients from undergoing treatment with SSRIs. Adverse effects of the TCAs include dry mouth, constipation, blurred vision antihistaminergic effects, sedation, and weight gain. MAOIs are rarely used, since this class of drug often has interactions with numerous other drugs and many foods. The most common side effects of MAOIs include decrease in blood pressure when standing up and insomnia. Lithium in excess is toxic, and other side effects include dizziness, diarrhea, drowsiness, nausea tremors and weight gain. TCAs can interact with MAOIs to produce fever, agitation, convulsions and even death.

In addition, SSRI's have interactions with other antidepressants. For example, SSRIs can cause TCA levels to increase resulting in abnormal heart rhythms. Interactions of SSRIs with MAOIs is known to cause serious, sometimes fatal, reactions including hyperthermia, rigidity, myoclonus, autonomic instability with possible rapid fluctuations of vital signs, and mental status changes that include extreme agitation progressing to delirium and coma. Serotonin syndrome results from excessive stimulation of central and peripheral serotonergic receptors. Serotonin syndrome is a dangerous and potentially fatal syndrome which includes rapid changes in vital signs (fever, oscillations in blood pressure), sweating, nausea, vomiting, rigid muscles, myoclonus, agitation, delirium, seizures, and coma. Increased risk of seizures; caution required. SSRIs are not administered jointly due to increased SSRI blood levels.

In contrast to the above-described agents, NARIs have a totally different profile. They have almost no affinity for serotonin and dopamine uptake sites, or for adrenergic, histaminergic, and muscarinic receptors. Side effects from blocking norepinephrine reuptake and increasing its availability in the central and peripheral nervous system include tremor, tachycardia, and urinary hesitancy. Potential side effects of NARIs include nausea, headache, dry mouth, sedation, and tremors.

Most side effects are dose-dependent. Therefore, patients suffering from such side effects would benefit from administration of a lower dose of drug(s), if the lower dose could be rendered therapeutically effective.

### NMDA Receptors and Mood Disorders

The N-methyl-D-aspartate (NMDA) receptor is a postsynaptic, ionotropic receptor which is responsive to, *inter alia,* the excitatory amino acids glutamate and glycine and the synthetic compound NMDA, hence the receptor name. The NMDA receptor controls the flow of both divalent (Ca ++ ) and monovalent (Na+ , K+ ) ions into the postsynaptic neural cell through a receptor associated channel (Foster et al., Nature 1987; 329:395-396; Mayer et al., Trends in Pharmacol. Sci. 1990; 11:254-260). There is also a strychnine insensitive glycine binding site proximate to the NMDA/glutamate/aspartate binding site. Glycine is a co-agonist along with glutamate at the NMDA receptor complex and simultaneous binding of the two agonists is required for the activation of the NMDA receptor channel. Binding of partial glycine agonists to that site allosterically inhibits the NMDA binding site. Such partial glycine agonists are also considered to be NMDA antagonists.

Abnormal NMDA receptor activation resulting from, among other causes, excessive and/or sustained synaptic levels of the excitatory amino acid glutamate or oxidative stress leads to increased calcium influx. The high levels of intracellular calcium that are produced activate biochemical cascades that affect normal cell function, and, if maintained over long periods of time, results in protein, DNA and membrane degradation leading to cell damage and ultimately cell death. This phenomenon, known as excitotoxicity, is also thought to be responsible for the neurological damage associated with other disorders ranging from hypoglycemia and cardiac arrest, to epilepsy.

Excessive activation of glutamate receptors also has been implicated in anxiety and anxiety disorders. Significantly higher glutamate plasma levels have been detected in patients with mood disorders than in comparison subjects. In social interaction tests on rats, the blocking of basal glutamate excitation elicited anxiolytic-like effects (Brodkin et al., Pharmacol. Biochem. Behav. 2002; 73(2):359-66). In addition, there are reports indicating similar involvement in the chronic neurodegeneration of Huntington's, Parkinson's, and Alzheimer's diseases (Mattson, Neuromolecular Med. 2003; 3(2):65-94). Activation of the NMDA receptor has been shown to be responsible for post-stroke convulsions, and, in certain models of epilepsy, activation of the NMDA receptor has been shown to be necessary for the generation of seizures.

Drugs acting on the NMDA receptor are therefore expected to have significant therapeutic potential. For instance, U.S. Pat. No. 4,904,681, issued to Cordi et al. (Cordi I), describes the use of D-Cycloserine, which was known to modulate the NMDA receptor, to improve/enhance memory and to treat cognitive deficits linked to a neurological disorder. D-Cycloserine is described as a glycine agonist which binds to the strychnine-insensitive glycine receptor.

U.S. Pat. No. 5,061,721, issued to Cordi et al. (Cordi II), describes the use of a combination of D-cycloserine and D-alanine to treat Alzheimer's disease, age-associated memory impairment, learning deficits, and psychotic disorders, as well as to improve memory or learning in healthy individuals. D-alanine is administered in combination with D-cycloserine to reduce the side effects observed in clinical trials of D- cycloserine, mainly those due to its growth-inhibiting effect on bacteria resulting in depletion of natural intestinal flora. D-alanine reverses the growth-inhibiting effect of D-cycloserine on bacteria. It is also reported that D-cycloserine actually has partial agonist character.

Certain adamantane derivatives and related compounds are known to have NMDA receptor antagonist activity. See, e.g., U.S. Patent 6, 071,966; 6,034,134; and 5,061,703, all incorporated herein by reference.

U.S. Pat. No. 5,086,072, issued to Trullas et al., describes the use of 1-aminocyclopropanecarboxylic acid (ACPC), which was known to modulate the NMDA receptor as a partial agonist of the strychnine-insensitive glycine binding site, to treat mood disorders including major depression, bipolar disorder, dysthymia and seasonal effective disorder. It is also therein described that ACPC mimics the actions of clinically effective antidepressants in animal models. Again, in the examples provided, the compound was administered intraperitoneally.

WO 00/02551, to Mueller et al., describes compounds active at both the serotonin reuptake site and the N-methyl-D-aspartate receptor that can be used to treat different types of disorders such as depression, obsessive-compulsive disorders (OCD), sleep disorders, sexual dysfunction, and eating disorders. According to this PCT, potent activity at the serotonin reuptake site is favored (less than about 100 nM), while an intermediate activity at the NMDA receptor is favored (IC₅₀ of about 100 nM to 5µM).. Too potent an activity at the NMDA receptor is less preferred because of possible PCP-like side effects.

However, despite progress, upwards of 30% to 45% of depressed patients who are treated with antidepressants show only partial or no response. Even among patients who are considered responders, there may be residual symptoms of depression. The presence of residual symptoms has been associated with a poorer prognosis and higher risk of relapse. Refractory depression, or treatment-resistant depression, is a clinical entity, therefore identified only on the basis of a poor clinical outcome. Accordingly, there remains a need in the art to provide combination treatment for depression that maximizes therapeutic efficacy in all patient populations, while decreasing unwanted adverse side effects.

### SUMMARY OF THE INVENTION

The present invention provides a method for treating mood disorders, including depression, by administering a combination of a functional NMDA receptor antagonist with one of a selective serotonin reuptake inhibitor (SSRI), that is citalopram or escitalopram.

In a preferred embodiment, the NMDA receptor antagonist is memantine or neramexane and the SSRI is citalopram.

In another preferred embodiment, the NMDA receptor antagonist is memantine and the SSRI is escitalopram.

In another embodiment, the NMDA receptor antagonist is neramexane and the SSRI is citalopram.

In yet a further embodiment, the NMDA receptor antagonist is neramexane and the SSRI is escitalopram.

In one embodiment, the NMDA antagonist or the SSRI are administered at sub-optimal or sub-threshold effects, where administration of the combination potentiates the therapeutic effect.

In another embodiment, both the NMDA antagonist and the SSRI are administered at sub-optimal or sub-threshold effects, where administration of the combination potentiates or has a synergistic therapeutic effect.

The present invention also provides two active ingredient compositions comprising (i) a first amount of an NMDA receptor antagonist and (ii) a second amount of a selective serotonin reuptake inhibitor (SSRI) that is citalopram or escitalopram.

In one preferred embodiment, the compounds are formulated in the same composition.

In another embodiment, the compounds are formulated in separate compositions but are provided e.g., in a blister pack or other co-packaging suitable for conjoint (preferably concurrent, or quasi-concurrent and at least within the same day) administration.

### BRIEF DESCRIPTION OF THE DRAWINGS

**Figure 1.** Figure 1 shows the effects of neramexane on immobility (left panel) and locomoter activity (right panel) at doses from 0 to 20 mg/kg in C57BI mice. Anova F(4,52)=67.34, P<0.001 and F(3,41)=6.056; P<0.01, respectively. Symbols: * P<0.05; ** P<0.01; *** P<0.001 vs. vehicle.
**Figure 2.** Figure 2 shows the effects of imipramine on immobility (left panel) and locomotor activity (right panel) in C57/B1 mice. Anova F(3,44)=17.94, P<0.001 and F(2,27)=3.388, P<0.05, respectively. Symbols: * P<0.05; ** P<0.01; *** P<0.001 vs. vehicle.
**Figure 3.** Figure 3 shows effects of fluoxetine on immobility (left panel) and locomotor activity (right panel) in C57/B1 mice. Anova F(3,36)=17.186, P<0.001 and F(2,27)=1.42; NS, respectively. Symbols: * P<0.05; ** P<0.01; *** P<0.001 vs. vehicle.
**Figure 4.** Figure 4 shows effects of venlafaxine on immobility (left panel) and locomotor activity (right panel) in C57/B1 mice. Anova F(4,44)=30.02, P<0.001 and F(2,27)=10.36; P<0.001, respectively. Symbols: * P<0.05; *** P<0.001 vs. vehicle.
**Figure 5.** Figure 5 shows effects of interaction of neramexane 2.5 mg/kg with imipramine on immobility (left panel) and locomotor activity (right panel) in C57/B1 mice. Symbols: * P<0.05 vs. Vehicle; # P<0.05 vs. corresponding dose of IMI; § P<0.05 vs. corresponding dose of NER.
**Figure 6.** Figure 6 shows effects of effects of interaction of neramexane 20 mg/kg with imipramine on immobility (left panel) and locomotor activity (right panel) in C57/B1 mice. Symbols: * P<0.05 vs. Vehicle; # P<0.05 vs. corresponding dose of IMI; § P<0.05 vs. corresponding dose of NER.
**Figure 7.** Figure 7 shows effects of interaction of neramexane 2.5 mg/kg with fluoxetine on immobility (left panel) and locomotor activity (right panel) in C57/B1 mice. Symbols: * P<0.05 vs. Vehicle; # P<0.05 vs. corresponding dose of IMI; § P<0.05 vs. corresponding dose of NER.
**Figure 8.** Figure 8 shows effects of interaction of neramexane 20 mg/kg with fluoxetine on immobility (left panel) and locomotor activity (right panel) in C57/B1 mice. Symbols: * P<0.05 vs. Vehicle; # P<0.05 vs. corresponding dose of IMI; § P<0.05 vs. corresponding dose of NER.
**Figure 9.** Figure 9 shows Effects of interaction of neramexane 2.5 mg/kg with venlafaxine on immobility (left panel) and locomotor activity (right panel) in C57/B1 mice. Symbols: * P<0.05 vs. Vehicle; # P<0.05 vs. corresponding dose of IMI; § P<0.05 vs. corresponding dose of NER.
**Figure 10.** Figure 10 shows Effects of interaction of neramexane 20 mg/kg with venlafaxine on immobility (left panel) and locomotor activity (right panel) in C57/B1 mice. Symbols: * P<0.05 vs. vehicle; # P<0.05 vs. corresponding dose of IMI; § P<0.05 vs. corresponding dose of NER.

### DETAILED DESCRIPTION OF THE INVENTION

Approximately one-third of patients do not respond to SSRIs or other drugs administered as monotherapy. The present invention unexpectedly provides enhanced therapeutic effect over monotherapy for the treatment of depression and mood disorders by using a combination of (i) an NMDA receptor antagonist and (ii) a selective serotonin reuptake inhibitor (SSRI), a norepinephrine reuptake inhibitor (NARI), a dual SSRI/NARI (a SNRI), or a tricyclic antidepressant (TCA). These combinations provide an additive (synergistic) effect at lower effective doses of each compound compared to monotherapy. This is demonstrated below using an animal model of depression.

The present inventors have conceived and demonstrated for the first time that the clinical administration of an 1-aminocyclohexane derivative such as memantine or neramexane, in combination with an SSRI, NARI, SNRI, or TCA, is an unexpectedly valuable pharmacotherapeutic approach to the treatment of depression, including treatment-resistant depression, and other mood disorders.

The advantages of the present invention include the following: (i) to provide enhanced antidepressant activity (over monotherapy) produced by a combination of a functional NMDA receptor antagonist and an SSRI, NARI, SNRI or TCA. This combination will improve the therapeutic efficacy of both compounds above that achieved if either were administered alone; (ii) to provide an alternative therapy for patients who are unresponsive or partially responsive to antidepressant monotherapy; and (iii) to provide therapeutic efficacy at lower (*i.e.,* suboptimal or subthreshold doses), thereby decreasing side effects (discussed above) associated with either compound, and/or increasing the efficacy of either compound by synergistic or additive mechanisms.

### NMDA Receptor Antagonists

NMDA receptor antagonists potentially have a wide range of therapeutic applications in numerous CNS disorders, including acute neurodegeneration (e.g., associated with stroke and trauma), chronic neurodegeneration (e.g., associated with Parkinson's disease, AD, Huntington's disease, and amyotrophic lateral sclerosis [ALS]), epilepsy, drug dependence, depression, anxiety, and chronic pain (for reviews *see*: Parsons et al., Drug News Perspect., 1998, 11:523-533; Parsons *et al.,* 1999, *supra*; Jentsch and Roth, Neuropsychopharmacology, 1999, 20: 201-205; Doble, Therapie, 1995, 50: 319-337)

Functional inhibition of NMDA receptors can be achieved through actions at different recognition sites within the NMDA receptor complex, such as: the primary transmitter site (competitive), the phencyclidine site located inside the cation channel (uncompetitive), the polyamine modulatory site, and the strychnine-insensitive, co-agonistic glycine site (glycine B) (Parsons *et al.,* 1999, *supra*). As NMDA receptors also play a crucial physiological role in various forms of synaptic plasticity such as those involved in learning and memory (*see, e.g.,* Collingridge and Singer, Trends Pharmacol. Sci., 1990, 11:290-296), neuroprotective agents possessing high affinity for the NMDA receptors are likely to impair normal synaptic transmission and thereby cause numerous side effects. Indeed, many NMDA receptor antagonists identified to date produce highly undesirable side effects at doses within their putative therapeutic range. Thus, clinical trials failed to support good therapeutic utility due to numerous side effects for such NMDA receptor antagonists as dizocilpine ((+)MK-801;(+)-5-methyl-10,11-dihydro-5H-dibenzocyclohepten-5,10-imine maleate), Cerestat® (CNS-1102), Licostinel® (ACEA 1021), Selfotel® (CGS-19755), and D-CPP-ene (SDZ EAA 494; EAA-494-Leppik, Epilepsia, 1998, 39 (Suppl 5):2-6; Sveinbjornsdottir et al., Epilepsia, 1993, 34:493-521; SCRIP 2229/30, 1997, p. 21). The challenge in the field has therefore been to develop NMDA receptor antagonists that prevent the pathological activation of NMDA receptors but allow their physiological activity.

Within the meaning of the present invention, the term "NMDA antagonist drugs" is used to refer to drugs, that can suppress the normal triggering of NMDA receptor-mediated neuronal firings. Preferred NMDA antagonist drugs of the invention are 1-aminocyclohexane derivatives such as memantine and neramexane. These compounds also have 5HT3 antagonist activity and/or neuronal nicotinic receptor antagonist activity.

NMDA receptor antagonists useful for the present invention include the following:

Memantine (1-amino-3,5-dimethyl adamantane) is an analog of 1-aminocyclohexane (amantadine) and is disclosed in U.S. Patents No. 4,122,193; 4,273,774; 5,061,703; and 5,614,560. Neramexane (1-amino-1,3,3,5,5-pentamethylcyclohexane) is also a derivative of 1-aminocyclohexane, and is disclosed in U.S. Patent No. 6,034,134.

Memantine and neramexane, as well as some other 1-aminoalkyl-cyclohexanes are systemically-active noncompetitive NMDA receptor antagonists having moderate affinity for the receptor. They exhibit strong voltage dependent characteristics and fast blocking/unblocking kinetics (Parsons *et al.,* 1999, *supra*; Görtelmeyer et al., Arzneim-Forsch/Drug Res., 1992, 42:904-913; Winblad et al., Int. J. Geriat. Psychiatry, 1999, 14:135-146; Rogawski, Amino Acids, 2000, 19: 133-49; Danysz et al., Curr. Pharm. Des., 2002, 8:835-43; Jirgensons et. al., Eur. J. Med. Chem., 2000, 35: 555-565). These compounds dissociate from the NMDA receptor channels much more rapidly than the high affinity NMDA receptor antagonists such as (+)MK-801 and attenuate disruption of neuronal plasticity produced by tonic overstimulation of NMDA receptors. Due to their relatively low affinity for the receptor, strong voltage dependency and fast receptor unblocking kinetics, these compounds are essentially devoid of the side effects of other NMDA receptor antagonists at doses within the therapeutic range (Kornhuber et al., Eur. J. Pharmacol., 1991, 206:297-311). Indeed, memantine has been applied clinically for over 15 years showing good tolerability with the number of treated patients exceeding 200,000 (Parsons *et al.,* 1999, *supra*).

Memantine, neramexane and other 1-aminoalkylcyclohexanes have been suggested to be useful in alleviation of various progressive neurodegenerative disorders such as dementia in AD, Parkinson's disease, and spasticity (*see, e.g.,* U.S. Patents No. 5,061,703; 5,614,560, and 6,034,134; Parsons *et al.,* 1999, *supra*; Möbius, ADAD, 1999,13:S172-178; Danysz et al., Neurotox. Res., 2000, 2:85-97; Winblad and Poritis, Int. J. Geriatr. Psychiatry, 1999, 14:135-146; Görtelmeyer *et al.,* 1992, *supra*; Danysz et al., Curr. Pharm. Des., 2002, 8:835-843; Jirgensons et. al., Eur. J. Med. Chem., 2000, 35: 555-565). These diseases result from disturbances of glutamatergic transmission, *i.e.,* the excessive influx of calcium through NMDA receptor channels, leading to the destruction of brain cells in specific brain areas, leading to the impaired function and ultimately the destruction of brain cells (Choi, J. Neurobiol., 23: 1261-1276, 1992; Rothman and Olney, Trends Neurosci., 10: 299, 1987; Kemp et al., Trends Pharmacol. Sci., 8: 414, 1987). Chronic treatment of adult rats with memantine has been shown to enhance the induction of hippocampal long-term potentiation, increase the durability of synaptic plasticity, improve spatial memory abilities, and reverse the memory impairment produced by NMDA receptor agonists (Barnes et al., Eur. J. Neurosci., 1996; 8:65-571; Zajaczkowski et al., Neuropharm., 1997, 36:961-971). 1-Aminocyclohexane derivatives, and specifically memantine, have also been suggested to be useful in the treatment of AIDS dementia (U.S. Patent No. 5,506,231), neuropathic pain (U.S. Patent No. 5,334,618), cerebral ischemia (U.S. Patent No. 5,061,703), epilepsy, glaucoma, hepatic encephalopathy, multiple sclerosis, stroke, and tardive dyskinesia (Parsons *et al.,* 1999, *supra*). Furthermore, memantine and neramexane were shown to selectively block thermal hyperalgesia and mechanical allodynia in some models of chronic and neuropathic pain at doses that do not significantly affect motor activity.without obvious effects on motor reflexes. 1-aminocyclohexane derivatives were also demonstrated to possess immunomodulatory, antimalarial, anti-Borna virus, and anti-Hepatitis C activities (*see*, *e.g.,* U.S. Patent No. 6,034,134 and references cited therein).

1-aminocyclohexane derivatives such as memantine and neramexane (*see* U.S. Patent Application No. 09/597,102 and its corresponding international patent application PCT EP 01/06964 published as WO 01/98253 on December 27, 2001; U.S. Patent No. 6,034,134) have also been suggested to function via non-NMDA-mediated pathways. Thus, memantine was shown to inhibit 5HT3-mediated current (in the native N1E-115 and heterologous HEK-293 cells) and NMDA receptor-mediated currents (in rat hippocampal slices) with approximately equal affinity (Parsons *et al.,* 1999, *supra;* Rammes et al., 2001, Neurosci. Lett., 306:81-84). Like memantine, neramexane was also shown to inhibit 5-HT3 receptor-mediated currents (Rammes et al., 2001, Neurosci. Lett., 306:81-84). 5HT3 receptor antagonists are known to improve learning and memory in animals (Carli et al., 1997, Behav. Brain Res., 82:185-194; Reznik and Staubli, 1997, J. Neurophysiol., 77:517-521).

### Definitions

The 1-aminocyclohexane derivatives as NMDA antagonists for use in the invention can be represented by the general formula (I): 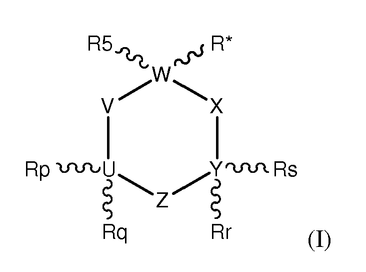 wherein:
R^{*} is -(A)ₙ-(CR¹R²)ₘ-NR³R⁴,
   n+m = 0, 1, or 2,
   A is selected from the group consisting of linear or branched lower alkyl (C₁-C₆),linear or branched lower alkenyl (C₂-C₆), and linear or branched lower alkynyl (C₂-C₆),
   R¹ and R² are independently selected from the group consisting of hydrogen, linear or branched lower alkyl (C₁-C₆), linear or branched lower alkenyl (C₂-C₆), linear or branched lower alkynyl (C₂-C₆) aryl, substituted aryl and arylalkyl,
   R³ and R⁴ are independently selected from the group consisting of hydrogen, linear or branched lower alkyl (C₁-C₆), linear or branched lower alkenyl (C₂-C₆), and linear or branched lower alkynyl (C₂-C₆), or together form alkylene (C₂-C₁₀) or alkenylene (C₂-C₁₀) or together with the N form a 3-7-membered azacycloalkane or azacycloalkene, including substituted (alkyl (C₁-C₆), alkenyl (C₂-C₆)) 3-7-membered azacycloalkane or azacycloalkene; or independently R³ or R⁴ may join with R^{p}, R^{q}, R^{r}, or R^{s} to form an alkylene chain -CH(R⁶)-(CH₂)ₜ,
   wherein t= 0 or 1 and the left side of the alkylene chain is attached to U or Y and the right side of the alkylene chain is attached to N and R⁶ is selected from the group consisting of hydrogen, linear or branched lower alkyl (C₁-C₆), linear or branched lower alkenyl (C₂-C₆), linear or branched lower alkynyl (C₂-C₆), aryl, substituted aryl and arylalkyl; or independently R³ or R⁴ may join with R⁵ to form an alkylene chain represented by the formula -CH₂-CH₂-CH₂-(CH₂)ₜ-, or an alkenylene chain represented by the formulae -CH=CH-CH₂-(CH₂)ₜ-, -CH=C=CH-(CH₂)ₜ- or -CH₂-CH=CH-(CH₂)ₜ-, wherein t= 0 or 1, and the left side of the alkylene or alkenylene chain is attached to W and the right side of the alkylene ring is attached to N;
R⁵ is independently selected from the group consisting of hydrogen, linear or branched lower alkyl (C₁-C₆), linear or branched lower alkenyl (C₂-C₆), and linear or branched lower alkynyl (C₂-C₆), or R⁵ combines with the carbon to which it is attached and the next adjacent ring carbon to form a double bond,
R^{p}, R^{q}, R^{r}, and R^{s}, are independently selected from the group consisting of hydrogen, linear or branched lower alkyl (C₁-C₆), linear or branched lower alkenyl (C₂-C₆), linear or branched lower alkynyl (C₂-C₆), cycloalkyl (C₃-C₆) and aryl, substituted aryl and arylaklyl or R^{p}, R^{q}, R^{r}, and R^{s} independently may form a double bond with U or with Y or to which it is attached, or R^{p}, R^{q}, R^{r}, and R^{s} may combine together to represent a lower alkylene -(CH₂)ₓ- or a lower alkenylene bridge wherein x is 2-5, inclusive, which alkylene bridge may, in turn, combine with R⁵ to form an additional lower alkylene -(CH₂)_{y}- or a lower alkenylene bridge, wherein y is 1-3, inclusive;
the symbols U, V, W, X, Y, Z represent carbon atoms, and include optical isomers, diastereomers, polymorphs, enantiomers, hydrates, pharmaceutically acceptable salts, and mixtures of compounds within formula (I).

The ring defined by U-V-W-X-Y-Z is preferably selected from the group consisting of cyclohexane, cyclohex-2-ene, cyclohex-3-ene, cyclohex-1,4-diene, cyclohex-1,5-diene, cyclohex-2,4-diene, and cyclohex-2,5-diene.

Various salts and isomers (including stereoisomers and enantiomers) of the NMDA antagonists can be used. The term "salts" can include acid addition salts or addition salts of free bases. Examples of acids which may be employed to form pharmaceutically acceptable acid addition salts include inorganic acids such as hydrochloric, sulfuric, or phosphoric acid, and organic acids such as acetic, maleic, succinic, or citric acid, etc. All of these salts (or other similar salts) may be prepared by conventional means. The nature of the salt or isomer is not critical, provided that it is non-toxic and does not substantially interfere with the desired pharmacological activity. A preferred salt for the method of the present invention is memantine hydrochloride. A second preferred salt is neramexane mesylate.

The term "1-aminocyclohexane derivative" is used herein to describe a compound which is derived from 1-aminocyclohexane (or an available derivative thereof, such as neramexane or memantine) in the process used to create a similar but slightly different drug.

The term "analog" or "derivative" is used herein in the conventional pharmaceutical sense, to refer to a molecule that structurally resembles a reference molecule (such as 1-aminocyclohexane), but has been modified in a targeted and controlled manner to replace one or more specific substituents of the referent molecule with an alternate substituent, thereby generating a molecule which is structurally similar to the reference molecule. Synthesis and screening of analogs (e.g., using structural and/or biochemical analysis), to identify slightly modified versions of a known compound which may have improved or biased traits (such as higher potency and/or selectivity at a specific targeted receptor type, greater ability to penetrate mammalian blood-brain barriers, fewer side effects, etc.) is a drug design approach that is well known in pharmaceutical chemistry.

Non-limiting examples of 1-aminocyclohexane derivatives used according to the invention include the 1-aminoalkylcyclohexane derivatives selected from the group consisting of:
1-amino-1,3,5-trimethylcyclohexane,
1-amino-1(trans),3(trans),5-trimethylcyclohexane,
1-amino-1(cis),3(cis),5-trimethylcyclohexane,
1-amino-1,3,3,5-tetramethylcyclohexane,
1-amino-1,3,3,5,5-pentamethylcyclohexane (neramexane),
1-amino-1,3,5,5-tetramethyl-3-ethylcyclohexane,
1-amino-1,5,5-trimethyl-3,3-diethylcyclohexane,
1-amino-1,5,5-trimethyl-cis-3-ethylcyclohexane,
1-amino-(1S,5S)cis-3-ethyl-1,5,5-trimethylcyclohexane,
1-amino-1,5,5-trimethyl-trans-3-ethylcyclohexane,
1-amino-(1R,5S)trans-3-ethyl-1,5,5-trimethylcyclohexane,
1-amino-1-ethyl-3,3,5,5-tetramethylcyclohexane,
1-amino-1-propyl-3,3,5,5-tetramethylcyclohexane,
N-methyl-1-amino-1,3,3,5,5-pentamethylcyclohexane,
N-ethyl-1-amino-1,3,3,5,5-pentamethyl-cyclohexane,
N-(1,3,3,5,5-pentamethylcyclohexyl)pyrrolidine,
3,3,5,5-tetramethylcyclohexylmethylamine,
1-amino-1-propyl-3,3,5,5-tetramethylcyclohexane,
1 amino-1,3,3,5(trans)-tetramethylcyclohexane (axial amino group),
3-propyl-1,3,5,5-tetramethylcyclohexylamine semihydrate,
1-amino-1,3,5,5-tetramethyl-3-ethylcyclohexane,
1-amino-1,3,5-trimethylcyclohexane,
1-amino-1,3-dimethyl-3-propylcyclohexane,
1-amino-1,3(trans),5(trans)-trimethyl-3(cis)-propylcyclohexane,
1-amino-1,3-dimethyl-3-ethylcyclohexane,
1-amino-1,3,3-trimethylcyclohexane,
cis-3-ethyl-1(trans)-3(trans)-5-trimethylcyclohexamine,
1-amino-1,3(trans)-dimethylcyclohexane,
1,3,3-trimethyl-5,5-dipropylcyclohexylamine,
1-amino-1-methyl-3(trans)-propylcyclohexane,
1-methyl-3 (cis)-propylcyclohexylamine,
1-amino-1-methyl-3(trans)-ethylcyclohexane,
1-amino-1,3,3-trimethyl-5(cis)-ethylcyclohexane,
1-amino-1,3,3-trimethyl-5(trans)-ethylcyclohexane,
cis-3-propyl-1,5,5-trimethylcyclohexylamine,
trans-3-propyl-1,5,5-trimethylcyclohexylamine,
N-ethyl-1,3,3,5,5-pentamethylcyclohexylamine,
N-methyl-1-amino-1,3,3,5.5-pentamethylcyclohexane,
1-amino-1-methylcyclohexane,
N,N-dimethyl-1-amino-1,3,3,5,5-pentamethylcyclohexane,
2-(3,3,5,5-tetramethylcyclohexyl)ethylamine,
2-methyl-1-(3,3,5,5-tetramethylcyclohexyl)propyl-2-amine,
2-(1,3,3,5,5-pentamethylcyclohexyl-l)-ethylamine semihydrate,
N-(1,3,3,5,5-pentamethylcyclohexyl)-pyrrolidine,
1-amino-1,3(trans),5(trans)-trimethylcyclohexane,
1-amino-1,3(cis),5(cis)-trimethylcyclohexane,
1-amino-(1R,SS)trans-5-ethyl-1,3,3-trimethylcyclohexane,
1-amino-(1S,SS)cis-5-ethyl-1,3,3-trimethylcyclohexane,
1-amino-1,5,5-trimethyl-3(cis)-isopropyl-cyclohexane,
1-amino-1,5,5-trimethyl-3(trans)-isopropyl-cyclohexane,
1-amino-1-methyl-3(cis)-ethyl-cyclohexane,
1-amino-1-methyl-3(cis)-methyl-cyclohexane,
1-amino-5,5-diethyl-1,3,3-trimethyl-cyclohexane,
1-amino-1,3,3,5,5-pentamethylcyclohexane,
1-amino-1,5,5-trimethyl-3,3-diethylcyclohexane,
1-amino-1-ethyl-3.3,5,5-tetramethylcyclohexane,
N-ethyl-1-amino-1,3,3,5,5-pentamethylcyclohexane,
N-(1,3,5-trimethylcyclohexyl)pyrrolidine or piperidine,
N-[1,3(trans),5(trans)-trimethylcyclohexyl]pyrrolidine or piperidine,
N-[1,3(cis),5(cis)-trimethylcyclohexyl]pyrrolidine or piperidine,
N-(1,3,3,5-tetramethylcyclohexyl)pyrrolidine or piperidine,
N-(1,3,3,5,5-pentamethylcyclohexyl)pyrrolidine or piperidine,
N-(1,3,5,5-tetramethyl-3-ethylcyclohexyl)pyrrolidine or piperidine,
N-(1,5,5-trimethyl-3,3-diethylcyclohexyl)pyrrolidine or piperidine,
N-(1,3,3-trimethyl-cis-5-ethylcyclohexyl)pyrrolidine or piperidine,
N-[(1S,SS)cis-5-ethyl-1,3,3-trimethylcyclohexyl]pyrrolidine or piperidine,
N-(1,3,3-trimethyl-trans-5-ethylcyclohexyl)pyrrolidine or piperidine,
N-[(1R,SS)trans-5-ethyl,3,3-trimethylcyclohexyl]pyrrolidine or piperidine,
N-(1-ethyl-3,3,5,5-tetramethylyclohexyl)pyrrolidine or piperidine,
N-(1-propyl-3,3,5,5-tetramethylcyclohexyl)pyrrolidine or piperidine,
N-(1,3,3,5,5-pentamethylcyclohexyl)pyrrolidine,
their optical isomers, diastereomers, enantiomers, hydrates, their pharmaceutically acceptable salts, and mixtures thereof.

Certain 1-aminocyclohexane derivatives of general formula (I) including the case where three axial alkyl substituent, *e.g.,* R^{p}, R^{r} and R⁵ all together form a bridgehead to yield compounds (so called 1-aminoadamantanes) illustrated by the formulae IIb - IId below: 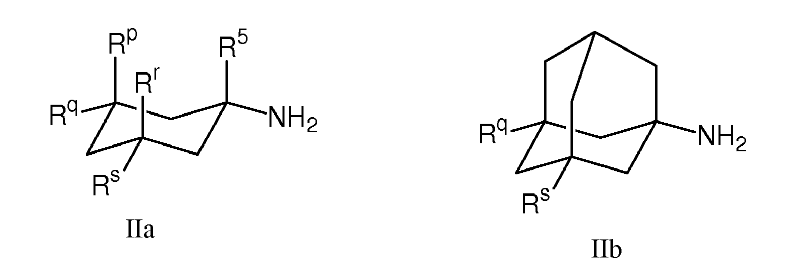 or

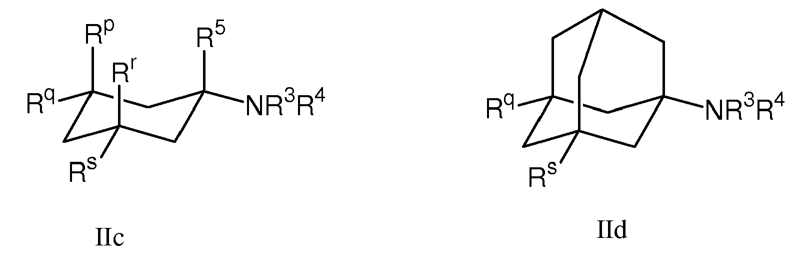

Certain 1-aminocyclohexane derivatives of formula (I) wherein n + m = 0, U, V, W, X, Y and Z form a cyclohexane ring, and one or both of R³ and R⁴ are independently joined to said cyclohexane ring via alkylene bridges formed through R^{p}, R^{q}, R^{r}, R^{s} or R⁵ are represented by the following formulae IIIa-IIIc: 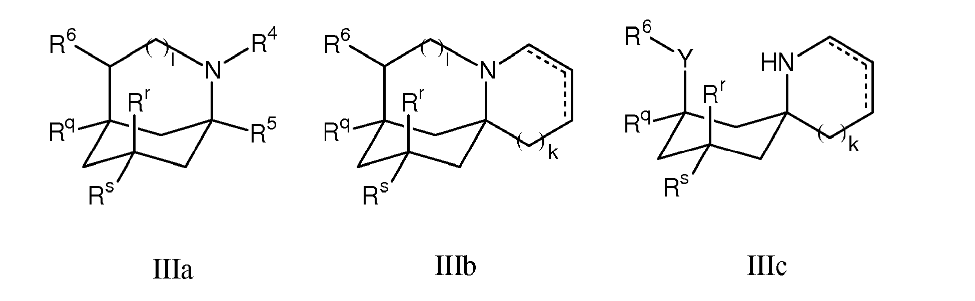 where R^{q} R^{r}, R^{s}, R^{r} and R⁵ are as defined above for formula (I), R⁶ is hydrogen, linear or branched lower alkyl (C₁-C₆), linear or branched lower alkenyl (C₂-C₆), linear or branched lower alkynyl (C₂-C₆), aryl, substituted aryl or arylalkyl Y is saturated or may combine with R⁶ to form a carbon-hydrogen bond with the ring carbon to which it is attached, 1= 0 or 1 and k= 0, 1 or 2 and ------ represents a single or double bond.

Non-limiting examples of 1-aminocyclohexane derivatives used according to the invention include 1-amino adamantane and its derivatives selected from the group consisting of:
1-amino-3-phenyl adamantane,
1-amino-methyl adamantane,
1-amino-3,5-dimethyl adamantane (memantine),
1-amino-3-ethyl adamantane,
1-amino-3-isopropyl adamantane,
1-amino-3-n-butyl adamantane,
1-amino-3,5-diethyl adamantane,
1-amino-3,5-diisopropyl adamantane,
1-amino-3,5-di-n-butyl adamantane,
1-amino-3-methyl-5-ethyl adamantane,
1-N-methylamino-3,5-dimethyl adamantane,
1-N-ethylamino-3,5-dimethyl adamantane,
1-N-isopropyl-amino-3,5-dimethyl adamantane,
1-N,N-dimethyl-amino-3,5-dimethyl adamantane,
1-N-methyl-N-isopropyl-amino-3-methyl-5-ethyl adamantane,
1-amino-3-butyl-5-phenyl adamantane,
1-amino-3-pentyl adamantane,
1-amino-3,5-dipentyl adamantane,
1-amino-3-pentyl-5-hexyl adamantane,
1-amino-3-pentyl-5-cyclohexyl adamantane,
1-amino-3-pentyl-5-phenyl adamantane,
1-amino-3-hexyl adamantane,
1-amino-3,5-dihexyl adamantane,
1-amino-3-hexyl-5-cyclohexyl adamantane,
1-amino-3-hexyl-5-phenyl adamantane,
1-amino-3-cyclohexyl adamantane,
1-amino-3,5-dicyclohexyl adamantane,
1-amino-3-cyclohexyl-5-phenyl adamantane,
1-amino-3,5-diphenyl adamantane,
1-amino-3,5,7-trimethyl adamantane,
1-amino-3,5-dimethyl-7-ethyl adamantane,
1-amino-3,5-diethyl-7-methyl adamantane,
1-N-pyrrolidino and 1-N-piperidine derivatives,
1-amino-3-methyl-5-propyl adamantane,
1-amino-3-methyl-5-butyl adamantane,
1-amino-3-methyl-5-pentyl adamantane,
1-amino-3-methyl-5-hexyl adamantane,
1-amino-3-methyl-5-cyclohexyl adamantane,
1-amino-3-methyl-5-phenyl adamantane,
1-amino-3-ethyl-5-propyl adamantane,
1-amino-3-ethyl-5-butyl adamantane,
1-amino-3-ethyl-5-pentyl adamantane,
1-amino-3-ethyl-5-hexyl adamantane,
1-amino-3-ethyl-5-cyclohexyl adamantane,
1-amino-3-ethyl-5-phenyl adamantane,
1-amino-3-propyl-5-butyl adamantane,
1-amino-3-propyl-5-pentyl adamantane,
1-amino-3-propyl-5-hexyl adamantane,
1-amino-3-propyl-5-cyclohexyl adamantane,
1-amino-3-propyl-5-phenyl adamantane,
1-amino-3-butyl-5-pentyl adamantane,
1-amino-3-butyl-5-hexyl adamantane, 1-amino-3-butyl-5-cyclohexyl adamantane,
their optical isomers, diastereomers, enantiomers, hydrates, N-methyl, N,N-dimethyl, N-ethyl, N-propyl derivatives, their pharmaceutically acceptable salts, and mixtures thereof.

The 1-amino adamantane derivatives of formulae IIb and IId, including memantine, are generally prepared by alkylation of halogenated adamantanes, preferably bromo- or chloroadamantanes. The di- or tri-substituted adamantanes are obtained by additional halogenation and alkylation procedures. The amino group is introduced either by oxidation with chromiumtrioxide and bromination with HBr or bromination with bromine and reaction with formamide followed by hydrolysis. The amino function can be alkylated according to generally-accepted methods. Methylation can, for example, be effected by reaction with chloromethyl formate and subsequent reduction. The ethyl group can be introduced by reduction of the respective acetamide. For more details on synthesis see, e.g., U.S. Patents No. 5,061,703 and 6,034,134. Additional synthetic techniques for the foregoing compounds can be found in provisional applications Ser. No. 60/350,974 filed November 7, 2001, Ser. No. 60/337,858 filed November 8, 2001, and Ser. No. 60/366,386 filed March 21, 2002, all incorporated by reference.

According to the invention, the 1-aminocyclohexane derivatives of formula (I) may be applied as such or used in the form of their pharmaceutically-acceptable salts including, for example, the acid addition salts such as hydrochlorides, hydrobromides, sulfates, acetates, succinates or tartrates, or their acid addition salts with fumaric, maleic, citric, or phosphoric acids.

In addition, using methods known to those skilled in the art, analogs and derivatives of the compounds of the invention can be created which have improved therapeutic efficacy in controlling depression or other mood disorders, *i.e.,* higher potency and/or selectivity at a specific targeted receptor type, either greater or lower ability to penetrate mammalian blood-brain barriers (e.g., either higher or lower blood-brain barrier permeation rate), fewer side effects, etc.

The term "mood disorder" includes major depression, bipolar disorder, unipolar depression, dysthymia, cyclothemia, seasonal affective disorder, and post-partum depression. The term mood disorder also refers to secondary depression resulting from a systemic or neurological disease. Examples of neurologic diseases include multiple sclerosis, Parkinson's disease, Alzheimer's disease, head trauma, cerebral tumors, post-stroke, early dementia, and sleep apnea, while systemic diseases include, but are not limited to infections, endocrine disorders, collagen vascular diseases, nutritional deficiencies and neoplastic disease. Secondary depression is also common in post-myocardial infarct patients, who exhibit a mortality three times that of non-depressed post-myocardial patients.

As used herein, the term "depression" refers to a clinical syndrome that includes a persistent sad mood or loss of interest in activities, which lasts for at least two weeks in the absence of treatment. The Diagnostic and Statistical Manual of Mental Disorders (DSM-IV-TR) criteria can be used to diagnose patients as suffering from depression (American Psychiatric Association. Diagnostic and Statistical Manual of Mental Disorders-Text Revision. 4th ed. Washington: American Psychiatric Association; 2000). Similarly, the International Classification of Disease, version 10 (IDC-10), of the World Health Organization, lists criteria for depression.

The term depression contemplates all diseases and conditions which are associated with depression including those classified in the IDC-10 and DSM-IV rating scales. Such diseases or disorders comprise major depression, dysthymic disorder, depressive episodes of bipolar disorders and depressive episodes associated with other mood disorders, including seasonal mood disorders such as seasonal affective disorder, subsyndromal depression, single episode depression, post-partum depression, and mood disorders due to a general medical condition, substance induced mood disorder, recurrent or treatment-resistant depression, child abuse induced depression, atypical depression, cyclothymia, menstrual-related dysphoria, depression associated with somatoform disorder, and treatment-resistant depression.

Major depressive disorder and dysthymic disorder are differentiated based on chronicity, severity and persistence. In major depression the depressed mood must be present for two weeks. In dysthymic disorder the depressed mood must be present most days over a period of two (2) years. Usually major depressive disorder is characterized by its sharp contrast to usual functioning. A person with a major depressive episode can be functioning and feeling normally and suddenly develop severe symptoms of depression. By contrast a person with dysthymic disorder has chronic depression with less severe symptoms than major depression.

The "tricyclic antidepressants" or "TCAs" include primary, secondary, tertiary and substituted amines including amitriptyline, clomipramine, trimipramine, doxepin, nortriptyline, desipramine, and protriptyline.

The "selective serotonin reuptake inhibitors" or "SSRIs" include fluoxetine, paroxetine (Paxil®), citalopram (Celexa®), sertraline (Zoloft®) and fluvoxamine, and enantiomers thereof such as escitalopram (*i.e.,* Lexapro®). Preferred salts of these are fluoxetine hydrochloride, fluvoxamine maleate, paroxetine hydrochloride and hydrochloride hemihydrate, citalopram hydrobromide, escitalopram oxalate, and sertraline hydrochloride.

The "selective norepinephrine reuptake inhibitors" or "NARIs" include reboxetine and atomoxetine. More preferably, reboxetine mesylate and atomoxetine hydrochloride.

The dual "serotonin/norepinephrine reuptake inhibitors" or "SNRIs" include venlafaxine, milnacipran and duloxetine. More preferably, venlafaxine hydrochloride, milnacipran hydrochloride, and duloxetine hydrochloride.

Within the meaning of the present invention, the term "NMDA antagonist drugs" is used to refer to drugs that can attenuate NMDA receptor-mediated neuronal activity. Preferred NMDA antagonist drugs of the invention are 1-aminocyclohexane derivatives and analogs, including aminoadamantanes such as memantine and neramexane. An NMDA "functional antagonist" is any compound which possesses pharmaceutically efficacious properties in humans, and which reduces excessive activity at NMDA responsive cation channels. "Functional" antagonists according to the invention include compounds which are partial agonists of the strychnine-insensitive glycine binding site, as well as competitive and non-competitive antagonists at the NMDA receptor complex at other binding sites. This term also includes agents that modify the NMDA receptor in any way.

An NMDA "competitive antagonist" is a compound possessing competitive antagonist properties when compared with endogenous neurotransmitters glutamate and aspartate.

An NMDA "non-competitive antagonist" is a compound that reduces activity at NMDA-gated cation channels at loci other than the strychnine-insensitive glycine binding site or the NMDA binding site. An example of such site is within the cation channel, such as memantine. Non-competitive antagonists are preferred.

The term "treating", as used herein, refers to reversing, alleviating, inhibiting the progress of, or preventing overt symptomatic manifestations the disorder or condition to which such term applies, or one or more symptoms of such disorders or condition. The term "treatment", as used herein, refers to the act of treating, as "treating" is defined immediately above. For the present invention, the term treat means to alleviate or eliminate one or more of the symptoms of a recognized depressive disorder such as depression, bipolar disorder, or seasonal affective disorder.

The term "pharmaceutically effective amount", as used herein, refers to an amount of combination of drugs to alleviate or eliminate symptoms of a depressive disorder such as severe major depressive disorder; bipolar disorder; seasonal affective disorder, dysthymia, cyclothemia.

The term "combination" applied to active ingredients is used herein to define a single pharmaceutical composition (formulation) comprising both drugs of the invention (*i.e.,* an NMDA receptor antagonist and an SSRI, NARI, SNRI or TCA) or two separate pharmaceutical compositions (formulations), each comprising a single drug of the invention *(i.e.,* an NMDA receptor antagonist and an SSRI), to be administered conjointly.

The term "potentiate" means to increase the effect of, or act synergistically with, a drug or a biologic. In another embodiment, the term potentiate means that sub-optimal or sub-threshold amounts of individual compounds can be administered in combination to render a therapeutic effect which is about equal to or greater than the effect of the individual compound as monotherapy. In one embodiment of the present invention, the NMDA antagonist potentiates the therapeutic effect of an SSRI, an SNRI, an NARI, or a TCA. In another embodiment, the SSRI, SNRI, NARI, or TCA potentiates the therapeutic effect of the NMDA antagonist.

Within the meaning of the present invention, the term "conjoint administration" is used to refer to administration of the 1-aminocyclohexane derivative and an SSRI, NARI, SNRI, or TCA simultaneously in one composition, or simultaneously in different compositions, or sequentially. For the sequential administration to be considered "conjoint", however, the 1-aminocyclohexane derivative and SSRI, NARI, SNRI, or TCA must be administered separated by a time interval that still permits the resultant beneficial effect for treating, preventing, arresting a symptom or behavior associated with depression or another mood disorder in a mammal. For example, the 1-aminocyclohexane derivative and SSRI, NARI, SNRI, or TCA must be administered on the same day (e.g., each - once or twice daily), preferably within an hour of each other, and most preferably simultaneously.

The term "therapeutically effective" applied to dose or amount refers to that quantity of a compound or pharmaceutical composition that is sufficient to result in a desired activity upon administration to a mammal in need thereof. As used herein with respect to the pharmaceutical compositions comprising an 1-aminocyclohexane derivative, e.g., memantine or neramexane, and an SSRI, e.g., citalopram or escitalopram, NARI, SNRI or TCA, the term "therapeutically effective amount/dose" refers to the amount/dose of each compound that, when combined, is sufficient to produce an effective response upon administration to a mammal.

The term "sub-threshold" refers to the amount of an active ingredient inadequate to produce a response, *i.e.,* an amount below the minimum effective amount when the active ingredient is used as monotherapy.

The term "sub-optimal" in the same context means an amount of an active ingredient that produces a response but not to its full extent, which would be achieved with a higher amount.

The term "additive" refers to the combined effect of administering two compounds, where the overall response is equal to, or nearly equal to the sum of the responses if the compounds were administered as monotherapy.

The term "synergistic" refers to the combined effect of administering two therapeutic compounds where the overall response is greater than the sum of the two individual effects. The term synergy also refers to the combined effect of administering an amount of one compound that, when administered as monotherapy, produces no measurable response but, when administered in combination with another therapeutic compound, produces an overall response that is greater than that produced by the second compound alone.

The phrase "pharmaceutically acceptable", as used in connection with compositions of the invention, refers to molecular entities and other ingredients of such compositions that are physiologically tolerable and do not typically produce untoward reactions when administered to a mammal (e.g., human). Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans.

The term "carrier" applied to pharmaceutical compositions of the invention refers to a diluent, excipient, or vehicle with which an active compound (e.g., an 1-aminocyclohexane derivative or SSRI, NARI, SNRI or TCA) is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington's Pharmaceutical Sciences" by E.W. Martin, 18th Edition.

The term "subject" as used herein refers to a mammal. In particular, the term refers to humans presenting with depression or a mood disorder as discussed in the Background Section, above.

The term "about" or "approximately" usually means within 20%, more preferably within 10%, and most preferably still within 5% of a given value or range. Alternatively, especially in biological systems, the term "about" means within about a log (*i.e.,* an order of magnitude) preferably within a factor of two of a given value.

### Pharmaceutical Formulations and Administration

In conjunction with the methods of the present invention, also provided are pharmaceutical compositions comprising a therapeutically effective amount of an NMDA receptor antagonist (such as memantine or neramexane) and/or a therapeutically effective amount of an SSRI, NARI, SNRI or TCA, (such as escitalopram) as well as, optionally, an additional carrier or excipient (all pharmaceutically acceptable). Said compounds can be either formulated as a single composition or as two separate compositions, which can be administered conjointly (within the same day, preferably within an hour of each other and most preferably, simultaneously). Preferably, they are formulated as a single composition or as two separate compositions, which are preferably administered simultaneously. The compositions can be formulated for once-a-day administration or twice-a-day administration. Thus, the NMDA receptor antagonist b-i-d and the SSRI, NARI, SNRI or TCA can be administered b-i-d as one or as two different compositions for each administration. Or the two can be administered once a day (or vice-versa). Or they can each be administered once a day as one or as two different compositions.

In the disclosed compositions, both the NMDA receptor antagonist and the SSRI, NARI, SNRI or TCA may be present in therapeutically effective amounts, or one or both of them may be in a suboptimal or subthreshold amount. The optimal therapeutically effective amount should be determined experimentally, taking into consideration the exact mode of administration, form in which the drug is administered, the indication toward which the administration is directed, the subject involved (e.g., body weight, health, age, sex, responsiveness to a particular therapeutic agent, etc.), and the preference and experience of the physician or veterinarian in charge. As described herein, since there is a synergistic effect of the two compounds, one or both will be effective in the combination at an amount lower than its corresponding effective amount as monotherapy.

The compounds may be administered orally, topically, parenterally, or mucosally (e.g., buccally, by inhalation, or rectally) in dosage unit formulations containing conventional non-toxic pharmaceutically acceptable carriers. It is usually desirable to use the oral route. The compounds may be administered orally in the form of a capsule, a tablet, or the like, or as a semi-solid or liquid formulation (*see* Remington's Pharmaceutical Sciences, Mack 5 Publishing Co., Easton, PA). The orally administered medicaments may also be administered in the form of a time-controlled release vehicle, including diffusion-controlled systems, osmotic devices, dissolution-controlled matrices, and erodible/degradable matrices.

For oral administration in the form of a tablet or capsule, the compounds can be combined with a non-toxic, pharmaceutically acceptable excipients such as binding agents (e.g., pregelatinized maize starch, polyvinylpyrrolidone or hydroxypropyl methylcellulose); fillers (e.g., lactose, sucrose, glucose, mannitol, sorbitol and other reducing and non-reducing sugars, microcrystalline cellulose, calcium sulfate, or calcium hydrogen phosphate); lubricants (e.g., magnesium stearate, talc, or silica, steric acid, sodium stearyl fumarate, glyceryl behenate, calcium stearate, and the like); disintegrants (e.g., potato starch or sodium starch glycolate); or wetting agents (e.g., sodium lauryl sulphate), coloring and flavoring agents, gelatin, sweeteners, natural and synthetic gums (such as acacia, tragacanth or alginates), buffer salts, carboxymethylcellulose, polyethyleneglycol, waxes, and the like.

The tablets can be coated by methods well known in the art. The cores may also be coated with a concentrated sugar solution which may contain e.g., gum arabic, gelatine, talcum, titanium dioxide, and the like. Alternatively, the tablets can be coated with a polymer known to a person skilled in the art, wherein the polymer is dissolved in a readily volatile organic solvent or mixture of organic solvents.

For the formulation of soft gelatin capsules, the active substances may be admixed with e.g., a vegetable oil or poly-ethylene glycol. Hard gelatin capsules may contain granules of the active substances using either the above mentioned excipients for tablets e.g., lactose, saccharose, sorbitol, mannitol, starches (e.g., potato starch, corn starch or amylopectin), cellulose derivatives or gelatine. Also liquids or semisolids of the drug can be filled into hard gelatine capsules.

The compositions can be also introduced in microspheres or microcapsules, *e.g.,* fabricated from polyglycolic acid/lactic acid (PGLA) (see, *e.g.,* U.S. Patents No. 5,814,344; 5,100,669 and 4,849,222; PCT Publications No. WO 95/11010 and WO 93/07861). Biocompatible polymers useful in achieving controlled release of a drug, include for example, polylactic acid, polyglycolic acid, copolymers of polylactic and polyglycolic acid, polyepsilon caprolactone, polyhydroxybutyric acid, polyorthoesters, polyacetals, polyhydropyrans, polycyanoacrylates, and cross-linked or amphipathic block copolymers of hydrogels.

Formulation of the compounds in semi-solid or liquid form is within the skill of the art, as the active ingredient is highly soluble in aqueous media. Usually the active substance(s), e.g., memantine and an SSRI, will constitute between 0.1 and 99% by weight of the formulation, more specifically between 0.5 and 20% by weight for formulations intended for injection and between 0.2 and 50% by weight for formulations suitable for oral administration.

Liquid preparations for oral administration can take the form of, for example, solutions, syrups, emulsions or suspensions, or they can be presented as a dry product for reconstitution with water or other suitable vehicle before use. Preparations for oral administration can be suitably formulated to give controlled or postponed release of the active compound. A particular example of an oral time-controlled release pharmaceutical formulation is described in U.S. Patent No. 5,366,738.

For oral administration in liquid form, the compounds can be combined with non-toxic, pharmaceutically acceptable inert carriers (e.g., ethanol, glycerol, water), suspending agents (e.g., sorbitol syrup, cellulose derivatives or hydrogenated edible fats), emulsifying agents (e.g., lecithin or acacia), non-aqueous vehicles (e.g., almond oil, oily esters, ethyl alcohol or fractionated vegetable oils), preservatives (e.g., methyl or propyl-p-hydroxybenzoates or sorbic acid), and the like. Stabilizing agents such as antioxidants (BHA, BHT, propyl gallate, sodium ascorbate, citric acid) can also be added to stabilize the dosage forms. For example, solutions may contain from about 0.2% to about 20% by weight of memantine, with the balance being sugar and mixture of ethanol, water, glycerol and propylene glycol. Optionally such liquid formulations may contain coloring agents, flavoring agents, saccharine and carboxymethyl-cellulose as a thickening agent or other excipients known to a person skilled in the art.

For administration by inhalation, the compounds can be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide, or other suitable gas. In the case of a pressurized aerosol, the dosage unit can be determined by providing a valve to deliver a metered amount. Capsules and cartridges of, e.g., gelatin for use in an inhaler or insufflator can be formulated containing a powder mix of the compound and a suitable powder base such as lactose or starch.

Solutions for parenteral applications by injection can be prepared in an aqueous solution of a water-soluble pharmaceutically acceptable salt of the active substances, preferably in a concentration of from about 0.5% to about 10% by weight. These solutions may also contain stabilizing agents and/or buffering agents and may conveniently be provided in various dosage unit ampoules.

Dosage units for rectal application can be solutions or suspensions or can be prepared in the form of suppositories or retention enemas comprising the compounds in a mixture with a neutral fatty base, or gelatin rectal capsules comprising the active substances in admixture with vegetable oil or paraffin oil.

The formulations of the invention can be delivered parenterally, *i.e.,* by intravenous (i.v.), intracerebroventricular (i.c.v.), subcutaneous (s.c.), intraperitoneal (i.p.), intramuscular (i.m.), subdermal (s.d.), or intradermal (i.d.) administration, by direct injection, via, for example, bolus injection or continuous infusion. Formulations for injection can be presented in unit dosage form, e.g., in ampoules or in multi-dose containers, with an added preservative. Alternatively, the active ingredient can be in powder form for reconstitution with a suitable vehicle, e.g., sterile pyrogen-free water, before use.

The invention also provides a pharmaceutical pack or kit comprising one or more containers containing the NMDA antagonist and the SSRI, NARI, SNRI or TCA and those on the left representing ml units.

### Dosages

As disclosed herein, the dose of the components in the compositions of the present invention is determined to ensure that the dose administered continuously or intermittently will not exceed an amount determined after consideration of the results in test animals and the individual conditions of a patient. A specific dose naturally varies depending on the dosage procedure, the conditions of a patient or a subject animal such as age, body weight, sex, sensitivity, feed, dosage period, drugs used in combination, and seriousness of the disease. The appropriate dose and dosage times under certain conditions can be determined by the test based on the above-described indices but may be refined and ultimately decided according to the judgment of the practitioner and each patient's circumstances (age, general condition, severity of symptoms, sex, etc.) according to standard clinical techniques.

Toxicity and therapeutic efficacy of the compositions of the invention can be determined by standard pharmaceutical procedures in experimental animals, *e.g.,* by determining the LD₅₀ (the dose lethal to 50% of the population) and the ED₅₀ (the dose therapeutically effective in 50% of the population). The dose ratio between therapeutic and toxic effects is the therapeutic index and it can be expressed as the ratio ED₅₀/LD₅₀-Compositions that exhibit large therapeutic indices are preferred.

The drug combination of the invention is not only highly effective at relatively low doses but also possesses low toxicity and produces few side effects. Suitable daily doses of the active compounds in the combination of the invention in the therapeutic treatment of humans are about 0.01-10 mg/kg bodyweight on peroral administration and 0.001-10 mg/kg bodyweight on parenteral administration. The daily doses of the SSRI, NARI, SNRI or TCA may very well differ from the daily doses of the NMDA receptor antagonist, but the doses can also be the same for both of the active substances.

Specific amounts of the 1-aminocyclohexane derivative memantine which may be used in unit dosage amounts of the invention are in a range from about 1 mg to about 60 mg/day, and for neramexane, from about 10-100 mg/day. In one embodiment, memantine is administered in a range from about 5-20 mg/day and neramexane in a range from about 25-75 mg/day.

For the SSRIs, while the dosage depends on the drug, suitable doses can be from about 50-300 mg/day for fluvoxamine; 10-40 mg/day for fluoxetine, may increase to 80 mg/day; 50-200 mg/day for sertraline; 20-50 mg/day for paroxetine; 1-30 mg/day for a preferred embodiment, escitalopram; 10-60 mg/day for another preferred embodiment citalopram; and 75-200 mg/day buproprion.

Similarly, for the TCAs or other cyclic antidepressants, the dosage also depends on the drug. Suitable doses can be from about 150 mg/250 mg/day for imipramine; 25-300 mg/day for desipramine; between 50-75 mg per day, with some individuals requiring up to 150 mg, for nortryptiline; 150-300 mg/day of clomipramine; 200-300 mg/day of amitryptaline; 75-300 mg/day of doxepin; and 150-300 mg/day of venlafaxine.

In some particularly preferred embodiments, when the re-uptake inhibitor is citalopram, the amount to be administered to a human who will receive the combination of the present invention will be within the range from about 10 mg to about 60 mg per day, preferably, from about 20 to about 40 mg per day. When the re-uptake inhibitor is escitalopram, the amount to be administered to a human who will receive the combination of the invention will be within the range from about 1 to about 30 mg per day, preferably from about 5 to about 20 mg per day. When the NMDA receptor antagonist is memantine, the amount to be administered to a human who will receive the combination of the invention will be within the range from about 1 to 60 mg, preferably from about 5 to 20 mg. When the NMDA antagonist is neramexane, the range should be from about 10 to 100 mg, preferably from about 25 to 75 mg.

In a preferred embodiment, the amount of one or the other active ingredient or both is suboptimal or subthreshold and the effect is potentiated by the combination. For example, in one embodiment, where the NMDA antagonist is memantine and the SSRI is citalopram, memantine is administered at about 2.5 to 10 mg/day and citalopram at about 10 to 60 mg/day. Where the NMDA antagonist is memantine and the SSRI is escitalopram, the memantine is administered at about 2.5 to 10 mg/day and the escitalopram at about 1 to 30 mg/day. In another embodiment, where the NMDA antagonist is memantine and the SSRI is citalopram, the memantine is administered at about 1 to 60 mg/day and the citalopram at about 5 to 10 mg/day. Where the NMDA antagonist is memantine and the SSRI is escitalopram, the memantine is administered at about 1 to 60 mg/day and the escitalopram at about 2.5 to 5 mg/day.

In another embodiment where the NMDA antagonist is neramexane and the SSRI is citalopram, neramexane is administered at about 10 to 20 mg/day and citalopram at about 10 to 60 mg/day. Where the NMDA antagonist is neramexane and the SSRI is escitalopram, the neramexane is administered at about 10 to 20 mg/day and the escitalopram at about 1 to 30 mg/day. In another embodiment, where the NMDA antagonist is neramexane and the SSRI is citalopram, the neramexane is administered at about 10 to 100 mg/day and the citalopram at about 5 to 10 mg/day. Where the NMDA antagonist is neramexane and the SSRI is escitalopram, the neramexane is administered at about 10 to 100 mg/day and the citalopram at about 2.5 to 5 mg/day. As per the examples below, a sub-therapeutic dose of neramexane may be as low as 2.5 mg.

In another embodiment, both compounds are administered in sub-optimal or sub-threshold doses and the combination results in a synergistic therapeutic effect.

In another preferred embodiment, in the case of treatment resistant, less responsive or even refractory patients, the amount of each ingredient is sufficient to bring about an antidepressant effect as measured by at least one symptom or marker, upon the conjoint administration of the two active ingredients.

### EXAMPLES

The invention is also described by means of particular examples. However, the use of such examples anywhere in the specification is illustrative only and in no way limits the scope and meaning of the invention or of any exemplified term. Likewise, the invention is not limited to any particular preferred embodiments described herein. Indeed, many modifications and variations of the invention will be apparent to those skilled in the art upon reading this specification and can be made without departing from its spirit and scope. The invention is therefore to be limited only by the terms of the appended claims along with the full scope of equivalents to which the claims are entitled.

### Example 1: Forced Swimming Test Using a Combination of Memantine and Escitalopram

Monotherapy with an SSRI is typically found to be ineffective in the forced swim test, which is a model for depression. This model is, in part, representative of SSRI treatment-resistant depression (recurrent depression). Accordingly, the combination of an NMDA antagonist with SSRI antidepressants will be measured for efficacy in this model.

### Materials and Methods

***Animals.*** Male Wistar rats (250-270g) will be maintained at a constant temperature of 22EC, *ad libitum,* prior to drug administration. 10-12 rats per group will be used. Groups include a control (vehicle only), monotherapy (memantine or escitalopram only) and at least one group administered a combination of memantine and escitalopram (if more than one group, different amounts of each active ingredient will be used, including a suboptimal or subthreshold amount of each active ingredient). A "grid" illustrative of the different dosage amounts may be:

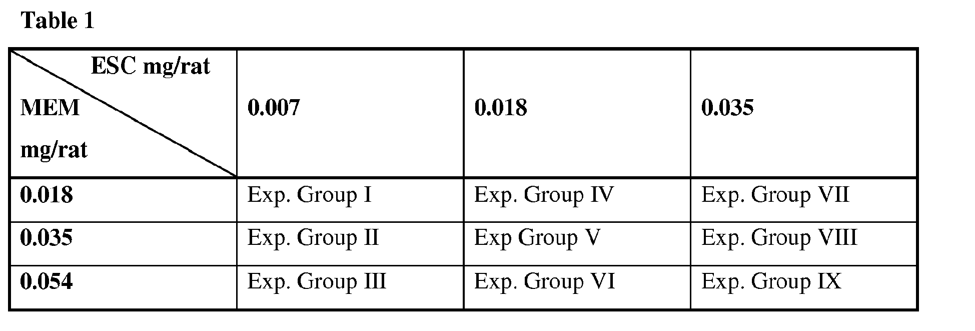

***Drugs.*** Memantine (1-amino-3,5-dimethyladamantane) is available from Merz Pharmaceuticals, Frankfurt, Germany, and Forest Laboratories, New York, NY. Escitalopram is available from Forest Laboratories, New York, NY and Lundbeck A/S, Copenhagen, Denmark. Drugs will be dissolved in a pharmaceutically acceptable carrier such as distilled water or saline (optionally with a preservative) for intraperitoneal injection. Administration will be one hour prior to experimentation. A range from about .001-10 mg/kg will be administered in 2-4 ml of aqueous carrier. For the combination group of memantine and escitalopram, the dosages of escitalopram will be will be from between 0.25-0.5 mg/kg and the dosage of memantine from about 2.5-5.0 mg/kg.

***Forced swimming test.*** This will be carried out according to the method described in Porsolt et al., Eur. J. Pharmacol. 1978; 27(4): 379-91, and modified by Detke et al., Psychopharmacology 1995; 121: 66-72. Briefly, rats are placed into water-filled cylinders such that the animal cannot have contact with they cylinder bottom. Using a time sampling technique to rate the predominant behaviors over 5 second intervals, three specific behavioral components of active behaviors are scored to evaluate antidepressant drug effects. These behaviors include climbing behavior, swimming behavior, and immobility. Compared with untreated or vehicle treated control animals, decreases in duration of immobility and increases in climbing or swimming behaviors are associated with the antidepressant-like activity of drug treatment.

### Results

It is expected that the combination of memantine and escitalopram will result in a significantly decreased duration of immobility and an increase in swimming or climbing behaviors, compared with the control, or when memantine or escitalopram are administered alone. It is also expected that this effect will be achieved with lower doses (*i.e.,* subthreshold doses if they were administered as monotherapy) of both memantine and escitalopram compared to each administered as monotherapy. For example, the same time difference might be observed with monotherapy, albeit at higher doses of either memantine or escitalopram than with the combination of both. In fact, one or both ingredients will be administered in a suboptimal or subthreshold amount.

### Example 2: Forced Swimming Test Using a Combination of Neramexane and Escitalopram

### Materials and Methods

The materials and methods will be substantially the same as above except for the replacement of memantine with neramexane (1-amino-1,3,3,5,5-pentamethyl-cyclohexan mesylate-also available from Merz Pharmaceuticals). A "grid" illustrative of the different dosage amounts may be:

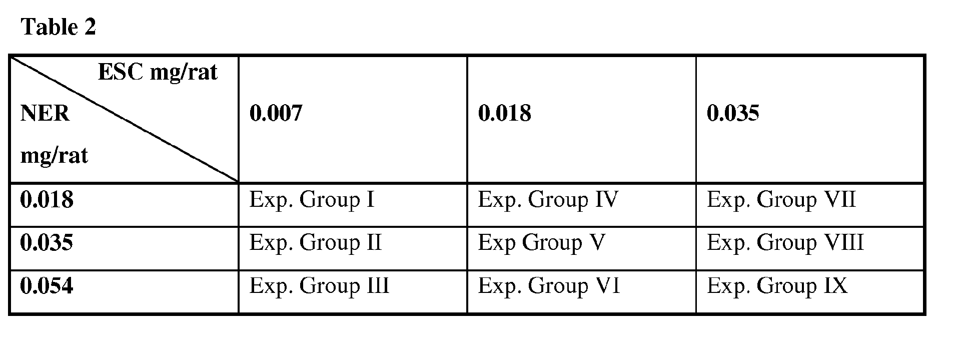

### Results

It is expected that the combination of neramexane and escitalopram will result in a significantly decreased duration of immobility and an increase in swimming or climbing behaviors, compared with the control, or when memantine or escitalopram are administered alone. It is also expected that this effect will be achieved with lower doses (*i.e.,* subthreshold doses if they were administered as monotherapy) of both neramexane and escitalopram compared to each administered as monotherapy. For example, the same time difference might be observed with monotherapy, albeit at higher doses of either neramexane or escitalopram than with the combination of both. In fact, one or both ingredients will be administered in a suboptimal or subthreshold amount.

### Example 3: Antidepressant Effects of Neramexane in Combination with an SSRI, SNRI, and TCA

The antidepressant-like activity of neramexane (NER), alone and in combination with three known antidepressant drugs (imipramine (IMI), fluoxetine (FLU) and venlafaxine (VEN) was investigated using the mouse tail suspension test (TST).

### Methods

Experiments were carried out essentially as described by Popik et al., Brit.J.Pharmacol. 2003; 139:1196-1202.

***Experimental Design.*** Dose-response analyses were performed for NER, IMI, FLU and VEN in the tail suspension test. A similar analysis was performed to evaluate nonspecific effects of the drugs on locomotor activity. Based on the TST activity of these compounds, two doses were chosen for the combination study: the lowest dose that produced a significant decrease of immobility (5 mg/kg in case of all antidepressants) and the dose that produced maximal or sub-maximal effect in this measure (20 mg/kg for all antidepressants). These doses were used to evaluate the antidepressant-like activity of the combination with 2.5 and 20 mg/kg of NER.

***Animals.*** Male C57/B1 mice obtained from IMP, Lodz, Poland and weighing about 25 g at the start of the experiment were employed in this study. Mice were group-housed in standard laboratory cages and kept in a temperature-controlled environment (21 ± 2°C) with a 12-hr light/dark cycle (light on: 07:00, off 19:00). Commercial food and tap water were available *ad libitum.* At least 12 mice per group were used in the TST study and 11 mice per group were used to assess the locomotor activity. Due to the shortage of animals, in some instances, mice that were used for the TST, were used one week later in the locomotor activity study. This is a commonly accepted procedure for this test.

***Drugs.*** Neramexane HCl (MRZ 2/579, Batch: "Ch.: P9-1475", "R 911840", MERZ), Imipramine HCl (Sigma, 1-7379, Lot 083K1037), Fluoxetine HCl (Tocris 0927, Batch 3A/39689) and Venlafaxine HCl (Forest Research Institute, Forest Lot # FMD-VEN-001, manufacturer: Cipla) were dissolved in sterile physiological saline (vehicle) assisted by a few minutes sonication. All drugs were administered in the volume of 10 ml/kg (0.2 ml/mouse), IP.

All experiments were carried out according to the National Institutes of Health Guide for Care and Use of Laboratory Animals (publication No. 85-23, revised 1985).

***Drug administration.*** TST Test: Drugs were administered 40 min (vehicle or NER) and 30 min (vehicle or antidepressants) before the TST. Immobility was induced by tail suspension according to the procedure of Steru et al. (Psychopharmacology 85:367-370, 1985). Mice were individually hung 75 cm above the flat surface with 1 cm broad paper adhesive tape placed about 1 cm from the tip of the tail. Immobility duration was recorded for 6 min. Three mice were observed simultaneously. Mice were considered immobile only when they hung passively and completely motionless. A trained observer, using a computer and the "PORSOLT" program, measured the duration of immobility. The experiments were always conducted in a blinded manner, i.e., with the experimenter unaware of the treatment conditions.

Locomotor Test. At least 1 hr before the start of experiment, mice were transferred to the experimental room for acclimation. Drugs were administered 40 min (vehicle or NER) and 30 min (vehicle or antidepressants) before the test. During the test, mice were placed in the aluminum actometers (custom made, cylindrical, 30 cm of diameter) and their gross movements were measured for 6 minutes. Mice were not adapted to the apparatus prior to evaluation.

***Evaluation of data.*** Immobility (in sec) and locomotor activity (arbitrary units/6 min) data were used for statistical analysis with the use of Statistica 5.0 for Windows.

For Part 1 (dose-response analysis for all compounds in the TST and locomotor activity), one-way ANOVAs followed by the Duncan's test were used. For the Part 2 (interaction between effects of various doses of NER and effects of various doses of antidepressants), two-way ANOVAs (with the dose of NER and dose of an antidepressant, as factors), and one-way ANOVAs (all doses, including vehicle) followed by Duncan's test were used. Data from vehicle treatment and data from some other treatments were pooled across several independent experiments to create single respective groups.

### Results

### A. Monotherapy

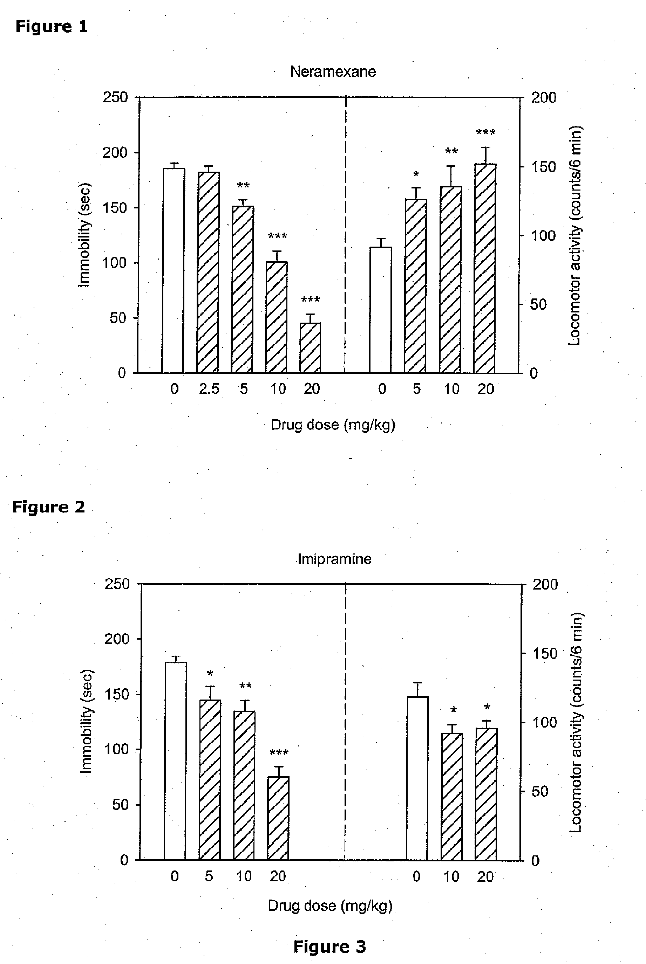

***Tail suspension test.*** All four compounds revealed dose-dependent antidepressant-like activity in the tail suspension test, with the MED of 5 mg/kg (Figures 1, 2, 3 and 4).

***Locomotor test.*** The same compounds, however, produced different effects on spontaneous locomotor activity. Thus, NER increased locomotor activity at all doses investigated (5, 10 and 20 mg/kg), IMI decreased it at all doses investigated (10 and 20 mg/kg), FLU did not change locomotor activity and VEN increased it at 40, but not 20 mg/kg. These data are presented on Figures 1, 2, 3 and 4.

### B. Combination with Imipramine

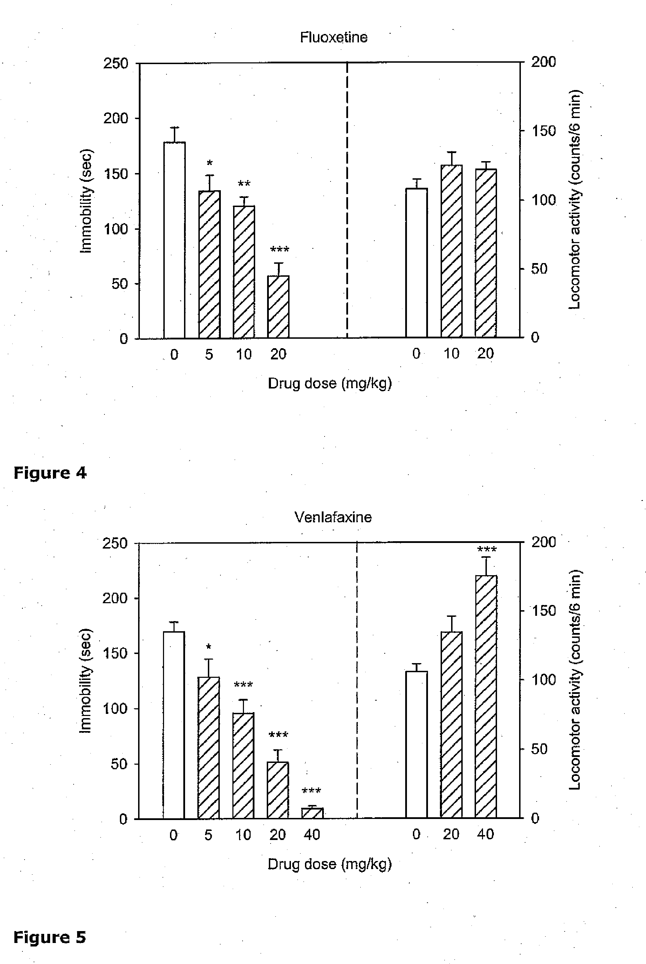

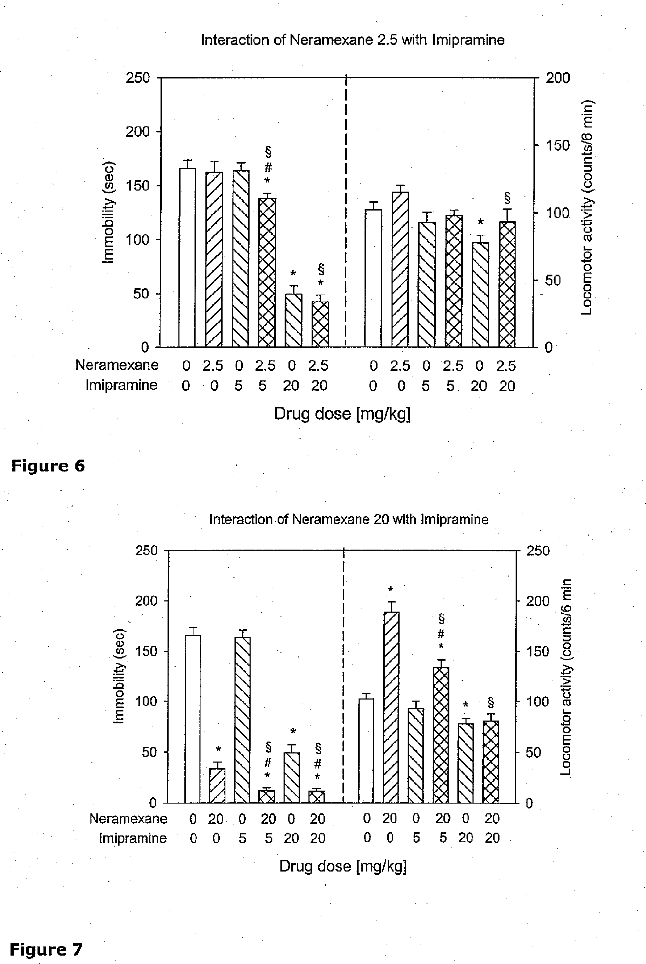

***Tail suspension test.*** When given with vehicle, NER at 20, but not 2.5 mg/kg decreased immobility. When given with vehicle, IMI at 20 but not 5 mg/kg also decreased immobility. The combination of NER 2.5 with IMI 5 mg/kg significantly decreased immobility. This effect was statistically significant from the effect of vehicle, NER at 2.5 mg/kg alone as well as IMI at 5 mg/kg alone (Figures 5 and 6).

Two-way ANOVA demonstrated significant effects of IMI dose, NER dose and interaction: F(2,98)=133.20; F(2,98)=222.48 and F(4,98)=25.01, respectively, P<0.001 in all cases.

***Locomotor test.*** When given with vehicle, NER at 20, but not 2.5 mg/kg increased locomotor activity. When given with vehicle, IMI at 20 but not 5 mg/kg decreased locomotor activity. The combination of NER 2.5 with IMI 5 mg/kg did not affect locomotor activity. However, the combination of NER 20 mg/kg with IMI 5 mg/kg or IMI 20 resulted in a marked decrease of locomotor activity as compared to NER used at 20 mg/kg (Figures 5 and 6).

Two-way ANOVA demonstrated significant effects of IMI dose, NER dose and interaction: F(2,90)=38.489; F(2,90)=29.76 and F(4,90)=11.78, respectively, P<0.001 in all cases.

### C. Combination with Fluoxetine

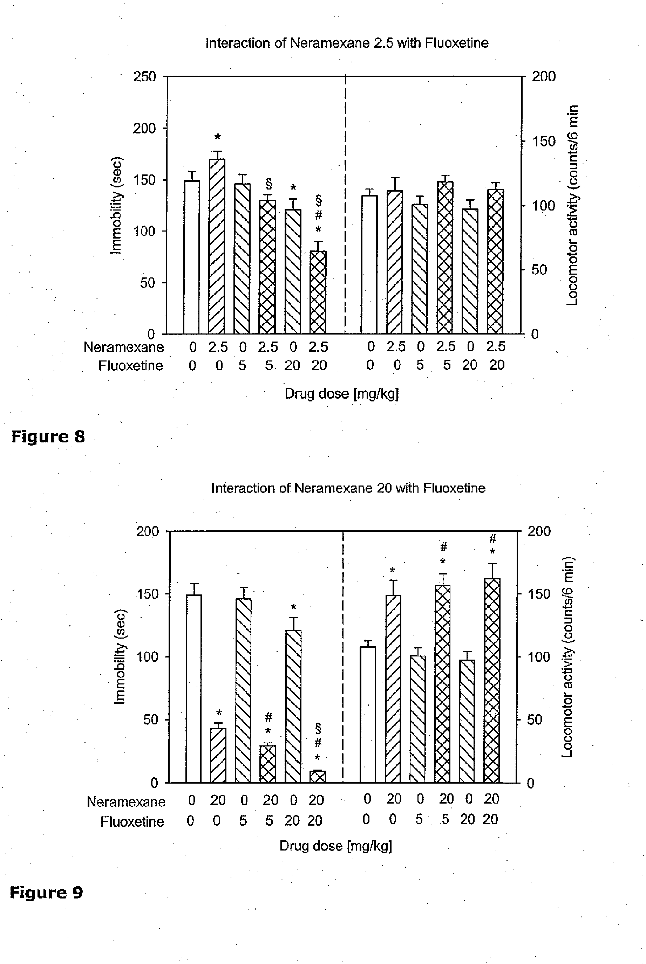

***Tail suspension test.*** When given with vehicle, NER at 20 mg/kg decreased, and, unexpectedly, at 2.5 mg/kg increased immobility. When given with vehicle, FLU at 20 but not 5 mg/kg decreased immobility. The combination of NER 2.5 with FLU 5 mg/kg did not change immobility as compared to vehicle, however it did reduce immobility as compared to NER at 2.5 mg/kg (but not FLU at 5 mg/kg alone), Figures 7 and 8.

Two-way ANOVA demonstrated significant effects of FLU dose, NER dose and interaction: F(2,95)=36.38; F(2,95)=205.0 and F(4,95)=5.53, respectively, P<0.001 in all cases.

***Locomotor test.*** When given with vehicle, NER at 20, but not 2.5 mg/kg increased locomotor activity. When given with vehicle, FLU did not affect locomotor activity. The combination of NER 2.5 with FLU 5 mg/kg also did not affect locomotor activity (Figures 7 and 8).

Two-way ANOVA demonstrated significant effects of NER dose F(2,88)=32.85; P<0.001 but not FLU dose F(2,88)=0.07; NS or interaction: F(4,88)=0.55; NS.

### D. Interaction with Venlafaxine

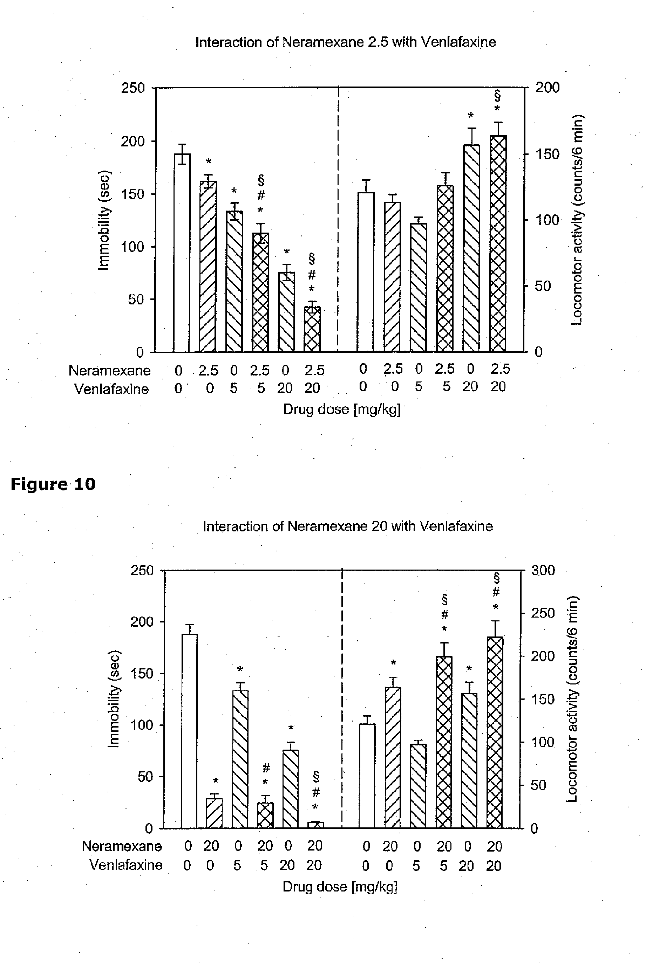

***Tail suspension test.*** When given with vehicle, NER decreased immobility at both 2.5 and 20 mg/kg. When given with vehicle, VEN at 5 and 20 mg/kg also decreased immobility. The combination of NER 2.5 with VEN 5 mg/kg significantly decreased immobility. This effect was statistically significant both from the effect of vehicle, NER at 2.5 alone as well as VEN at 5 mg/kg alone (Figures 9 and 10).

Two-way ANOVA demonstrated significant effects of VEN dose, NER dose and interaction: F(2,97)=112.23; F(2,97)=211.38 and F(4,97)=15.04, respectively, P<0.001 in all cases.

***Locomotor test.*** When given with vehicle, NER at 20, but not 2.5 mg/kg increased locomotor activity. When given with vehicle, VEN at 20 but not 5 mg/kg also increased locomotor activity. The combination of NER 2.5 with VEN 5 mg/kg did not affect locomotor activity. However, combination of NER 20 mg/kg with VEN 5 mg/kg or VEN at 20 mg/kg resulted in a marked increase of locomotor activity as compared to NER alone at 20 mg/kg (Figures 9 and 10).

Two-way ANOVA demonstrated significant effects of VEN dose and NER dose but no interaction: F(2,90)=13.97; F(2,90)=30.02 (P<0.001 in both cases) and F(4,90)=1.597; NS, respectively.

### Discussion

NER decreased the duration of immobility in the tail-suspension test at doses 5-20 mg/kg. In one out of three studies (Figure 7), NER (2.5 mg/kg) increased immobility time; in two out of three studies, this dose was without effect on immobility time, therefore NER-induced increase in immobility time is considered to be artifactual. IMI, FLU and VEN produced a decrease in the duration of immobility at doses 5-20 mg/kg.

Each of antidepressants combined with NER produced antidepressant-like effects with significant statistical interaction. Specifically, low and high doses of IMI potentiated effects of both low and high dose of NER. High dose of FLU potentiated effects of both low and high dose of NER. High dose of VEN potentiated effects of both low and high dose of NER, while the low dose of VEN potentiated effects of low but not high dose of NER.

**Table 3**

| | TST | | | | | | Locomotor activity | | | | | |
|---|---|---|---|---|---|---|---|---|---|---|---|---|
| | IMI₅ | IMI₂₀ | FLU₅ | FLU₂₀ | VEN₅ | VEN₂₀ | IMI₅ | IMI₂₀ | FLU₅ | FLU₂₀ | VEN₅ | VEN₂₀ |
| NER_{2.5} | ↑ | ↑ | ↑ | ↑ | ↑ | ↑ | - | - | - | - | - | ↑ |
| NER₂₀ | ↑ | ↑ | - | ↑ | - | ↑ | ↓ | ↓ | - | - | ↑ | ↑ |

In the locomotor activity studies, NER produced consistent locomotor stimulation at doses of 5-20 mg/kg, but not at 2.5 mg/kg. IMI at doses of 10-20 (but not 5) mg/kg decreased locomotor activity. FLU (5-20 mg/kg) generally did not affect locomotor activity. VEN at the dose of 40 mg/kg stimulated locomotion, this effect was also observed for the dose of 20 mg/kg in one of two studies (Fig 9 and 10); the dose of 5 mg/kg was without effect.

Each of antidepressants combined with NER, produced complex effects on locomotion. For IMI, but not FLU or VEN, these effects yield significant statistical interaction. Specifically, IMI at both low and high doses appeared to decrease the locomotor stimulatory effects of the high dose of NER. FLU did not influence the effects of NER on locomotion at any of the doses tested. With both doses of NER, a high dose of VEN appeared to produce a greater locomotor stimulatory effect than the respective doses of NER alone. Similar potentiation was observed when the high dose of VEN was used with a low dose of NER.

### Example 4: Evaluation of Memantine in Combination with Escitalopram in the Treatment of Depression in Alzheimer's Disease

To assess the safety and efficacy of memantine alone, and memantine in combination with escitalopram in patients with depression due to Alzheimer's disease (AD), the following study will be performed.

***Study design.*** The study will comprise an open-label, double-blind pilot study for 12 weeks with memantine (MEM), followed by 12 weeks of randomized double-blind treatment with either MEM plus placebo, or MEM plus escitalopram (ESC).

***Patient population.*** A total of 16 patients will be enrolled. Patients must be over 50 years old, and meet the NINCDS-ADRDA criteria for probable AD (confirmed with an MRI or CT scan), and the NIMH criteria for depression of AD. The patients must have a Mini-Mental State Examination (MMSE) score of at least 15 and not more than 24, and a Hamilton Depression Rating Scale Score of at least 18 at screening and baseline.

***Drug Treatment.*** During the first week of open-label treatment, patients will be administered 5 mg/day, up titrated to 10 mg/day b.i.d. at week two. Patients unable to tolerate 10 mg/day will be removed from the study. At the end of week 2, patients will increase to 15 mg/day memantine (3 times/day) for one week, increasing to 20 mg/day (4 times/day) at the beginning of week 4. The dose may be reduced to 15 mg/day or 10 mg/day in patients with dose-limiting events.

Patients will be maintained on 20 mg/day for weeks 5-8 and 9-12. Patients on lower doses may be re-challenged to the next higher dose.

For the remainder of the study (to week 24), patients will take the same dose as they were taking at the end of week 12. No dose adjustment will be permitted after week 12.

At the end of week 12, patients will be randomly assigned 10 mg of escitalopram or a placebo. The dose of memantine and escitalopram or placebo must be taken together (at the same time).

All doses are oral tablets.

**Evaluation.** Patients will be evaluated 10 times: at screening, baseline, and at the end of weeks 2, 4, 8, 12, 14, 16, 20 and 24 weeks. The following evaluations will be conducted at the indicated times:
MMSE: screening, baseline and the end of weeks 12 and 24, or upon early termination;
Cornell Scale for Depression in Dementia (CSDD): at each visit including early termination;
Clinical Global Impression (CGI): at each visit and early termination;
24-item Hamilton Depression Rating Scale (HAMD): at screening, baseline, the end of weeks 12 and 24, or upon early termination;
Montgomery Asberg Depression Rating Scale (MADRS): at baseline, the end of weeks 12 and 24, or upon early termination;
Alzheimer's Disease Assessment Scale-cognitive subscale (ADAS-cog): baseline, the end of weeks 12 and 24, or upon early termination; and
Alzheimer's Disease Cooperative Study-Activities of Daily Living inventory (ADCS-ADL): baseline, the end of weeks 12 and 24, or upon early termination.

### Results

It is expected that the combination of memantine and escitalopram will demonstrate increased efficacy on most if not all evaluation scales compared with memantine as monotherapy.

### Conclusion

These data demonstrates that the combination of an NMDA receptor antagonist and a SSRI, SNRI or TCA will increase the antidepressant efficacy of either compound, and may be effective in patients unresponsive to the antidepressant activity of either agent administered as monotherapy, or in patients unable to tolerate therapeutically effective amounts of monotherapy due to adverse effects. This effect is expected to be even more robust when the NMDA antagonist is memantine or neramexane and the second compound is an SSRI which is citalopram or escitalopram. Additionally, the potentiation produced with the combination allows for the administration of reduced doses of either or both compounds, thereby reducing adverse side effects.

The data also support efficacy for treating treatment-resistant (recurrent) depression. Although SSRIs are generally not effective for the treatment of treatment-resistant depression, it is expected that the combination of memantine or neramexane with citalopram and escitalopram, especially memantine and escitalopram, will be effective for treatment resistant depression.

All patent and literature references disclosed are herein incorporated in their entirety.

Key embodiments of the invention include the following:

In a first embodiment, the invention comprises a method for treating a mood disorder selected from the group consisting of depression, dysthymia, seasonal affective disorder, bipolar disorder and post-partum depression in a patient, which comprises administering to a patient in need thereof a combination comprising a therapeutically effective:
(i) first amount of a compound possessing functional antagonist properties for the N-methyl-D-aspartate (NMDA) receptor complex; and
(ii) second amount of a selective serotonin reuptake inhibitor (SSRI) that is citalopram or escitalopram, wherein combination of the first and the second amount is effective to treat the disorder.

In a second embodiment, the invention comprises the method of the first embodiment, wherein the combination produces a synergistic therapeutic effect.

In a third embodiment, the invention comprises the method of the first embodiment, wherein the N-methyl-D-aspartate (NMDA) receptor antagonist is a non-competitive NMDA receptor antagonist.

In a fourth embodiment, the invention comprises the method of the third embodiment, wherein the NMDA receptor antagonist is memantine or neramexane.

In a fifth embodiment, the invention comprises the method of the fourth embodiment, wherein the SSRI is citalopram and the NMDA receptor antagonist is memantine.

In a sixth embodiment, the invention comprises the method of the fourth embodiment, wherein the SSRI is escitalopram and the NMDA receptor antagonist is memantine.

In a seventh embodiment, the invention comprises the method of the fourth embodiment, wherein the SSRI is citalopram and the NMDA receptor antagonist is neramexane.

In an eighth embodiment, the invention comprises the method of the fourth embodiment, wherein the SSRI is escitalopram and the NMDA receptor antagonist is neramexane.

In a ninth embodiment, the invention comprises the method of the first embodiment, wherein at least one of the functional NMDA receptor antagonist and the SSRI compound of part (ii) are administered in a sub-threshhold amount.

In a tenth embodiment, the invention comprises the method of the first embodiment, wherein at least one of the functional NMDA receptor antagonist and the compound of part (ii) are administered in a sub-optimal amount.

In an eleventh embodiment, the invention comprises the method of the fifth embodiment, wherein the citalopram is administered at a dosage from about 10 to 60 mg/day and the memantine is administered from about 1 to 60 mg/day.

In a twelfth embodiment, the invention comprises the method of the eleventh embodiment, wherein the citalopram is administered at about 20 to 40 mg/day and the memantine is administered at about 5 to 20 mg/day

In a thirteenth embodiment, the invention comprises the method of the sixth embodiment, wherein the escitalopram is administered at a dosage from about 1 to 30 mg/day, and the memantine is administered at a dosage from about 1 to 60 mg/day.

In a fourteenth embodiment, the invention comprises the method of the thirteenth embodiment, wherein the escitalopram is administered at a dosage of about 5 to 20 mg/day, and the memantine is administered at a dosage from about 5 to 20 mg/day.

In a fifteenth embodiment, the invention comprises the method of the seventh embodiment, wherein the wherein the citalopram is administered at a dosage from about 10 to 60 mg/day and the neramexane is administered from about 10 to 100 mg/day.

In a sixteenth embodiment, the invention comprises the method of the fifteenth embodiment, wherein the citalopram is administered at about 20 to 40 mg/day and the neramexane is administered at about 25-75 mg/day

In a seventeenth embodiment, the invention comprises the method of the tenth embodiment, wherein the escitalopram is administered at a dosage from about 1 to 30 mg/day, and the neramexane is administered at a dosage from about 10 to 100 mg/day.

In an eighteenth embodiment, the invention comprises the method of the seventeenth embodiment, wherein the escitalopram is administered at a dosage of about 5 to 20 mg/day, and the neramexane is administered at a dosage from about 25 to 75 mg/day.

In a nineteenth embodiment, the invention comprises the method of the first embodiment, wherein administration of the combination of the NMDA antagonist and SSRI potentiates the therapeutic response compared to treatment of either compound as monotherapy.

In a twentieth embodiment, the invention comprises the method of the nineteenth embodiment, wherein the NMDA antagonist is memantine and is administered at about 2.5 to 10 mg/day and the SSRI is citalopram and is administered at about 10 to 60 mg/day.

In a twenty-first embodiment, the invention comprises the method of the nineteenth embodiment, wherein the NMDA antagonist is memantine and is administered at about 2.5 to 10 mg/kg second compound is escitalopram and is administered at about 1 to 30 mg/day.

In a twenty-second embodiment, the invention comprises the method of the nineteenth embodiment, wherein the NMDA antagonist is memantine and is administered at about 1 to 60 mg/day and the SSRI is citalopram and is administered at about 5 to 10 mg/day.

In a twenty-third embodiment, the invention comprises the method of the nineteenth embodiment, wherein the NMDA antagonist is memantine and is administered at about 1 to 60 mg/day and the SSRI is escitalopram and is administered at about 2.5 to 5 mg/day.

In a twenty-fourth embodiment, the invention comprises the method of the nineteenth embodiment, wherein the NMDA antagonist is neramexane and is administered at about 10 to 100 mg/day and the SSRI is citalopram and is administered at about 5 to 10 mg/day.

In a twenty-fifth embodiment, the invention comprises the method of the nineteenth embodiment, wherein the NMDA antagonist is neramexane and is administered at about 10 to 100 mg/day and the SSRI is escitalopram and is administered at about 2.5 to 5 mg/day.

In a twenty-sixth embodiment, the invention comprises the method of the nineteenth embodiment, wherein the NMDA antagonist is neramexane and is administered at about 10 to 20 mg/kg and the SSRI is citalopram and is administered at about 10 to 60 mg/day.

In a twenty-seventh embodiment, the invention comprises the method of the nineteenth embodiment, wherein the NMDA antagonist is neramexane and is administered at about 10 to 20 mg/kg and the SSRI is escitalopram and is administered at about 1 to 30 mg/day.

In a twenty-eighth embodiment, the invention comprises the method of the first embodiment, wherein the mood disorder is secondary depression resulting from a systemic or neurological disease.

In a twenty-ninth embodiment, the invention comprises the method of the twenty-eighth embodiment, wherein the neurological disease is multiple sclerosis, Parkinson's disease, Alzheimer's disease, head trauma, cerebral tumors, post-stroke, early dementia, or sleep apnea.

In a thirtieth embodiment, the invention comprises the method of the twenty-eighth embodiment, wherein the systemic disease is infection, endocrine disorder, collagen vascular disease, nutritional deficiency or neoplastic disease.

In a thirty-first embodiment, the invention comprises the method of the first embodiment, wherein the disorder is secondary depression in post-myocardial infarct patients.

In a thirty-second embodiment, the invention comprises the method of the first embodiment, wherein the functional NMDA antagonist has the following formula (I): 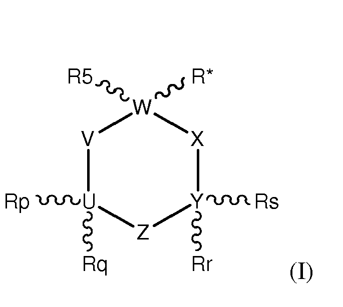 wherein:
R^{*} is -(A)ₙ-(CR¹R²)ₘ-NR³R⁴,
   n+m = 0, 1, or 2,
   A is selected from the group consisting of linear or branched lower alkyl (C₁-C₆),linear or branched lower alkenyl (C₂-C₆), and linear or branched lower alkynyl (C₂-C₆),
   R¹ and R² are independently selected from the group consisting of hydrogen, linear or branched lower alkyl (C₁-C₆), linear or branched lower alkenyl (C₂-C₆), linear or branched lower alkynyl (C₂-C₆) aryl, substituted aryl and arylalkyl,
   R³ and R⁴ are independently selected from the group consisting of hydrogen, linear or branched lower alkyl (C₁-C₆), linear or branched lower alkenyl (C₂-C₆), and linear or branched lower alkynyl (C₂-C₆), or together form alkylene (C₂-C₁₀) or alkenylene (C₂-C₁₀) or together with the N form a 3-7-membered azacycloalkane or azacycloalkene, including substituted (alkyl (C₁-C₆), alkenyl (C₂-C₆)) 3-7-membered azacycloalkane or azacycloalkene; or independently R³ or R⁴ may join with R^{p}, R^{q}, R^{r}, or R^{s} to form an alkylene chain -CH(R⁶)-(CH₂)_{t,}
   wherein t= 0 or 1 and the left side of the alkylene chain is attached to U or Y and the right side of the alkylene chain is attached to N and R⁶ is selected from the group consisting of hydrogen, linear or branched lower alkyl (C₁-C₆), linear or branched lower alkenyl (C₂-C₆), linear or branched lower alkynyl (C₂-C₆), aryl, substituted aryl and arylalkyl; or independently R³ or R⁴ may join with R⁵ to form an alkylene chain represented by the formula -CH₂-CH₂-CH₂-(CH₂)ₜ-, or an alkenylene chain represented by the formulae -CH=CH-CH₂-(CH₂)ₜ-, -CH=C=CH-(CH₂)ₜ- or -CH₂-CH=CH-(CH₂)ₜ-, wherein t= 0 or 1, and the left side of the alkylene or alkenylene chain is attached to W and the right side of the alkylene ring is attached to N;
R⁵ is independently selected from the group consisting of hydrogen, linear or branched lower alkyl (C₁-C₆), linear or branched lower alkenyl (C₂-C₆), and linear or branched lower alkynyl (C₂-C₆), or R⁵ combines with the carbon to which it is attached and the next adjacent ring carbon to form a double bond,
R^{p}, R^{q}, R^{r}, and R^{s}, are independently selected from the group consisting of hydrogen, linear or branched lower alkyl (C₁-C₆), linear or branched lower alkenyl (C₂-C₆), linear or branched lower alkynyl (C₂-C₆), cycloalkyl (C₃-C₆) and aryl, substituted aryl and arylaklyl or R^{p}, R^{q}, R^{r}, and R^{s} independently may form a double bond with U or with Y or to which it is attached, or R^{p}, R^{q}, R^{r}, and R^{s} may combine together to represent a lower alkylene -(CH₂)ₓ- or a lower alkenylene bridge wherein x is 2-5, inclusive, which alkylene bridge may, in turn, combine with R⁵ to form an additional lower alkylene -(CH₂)_{y}- or a lower alkenylene bridge, wherein y is 1-3, inclusive;
and wherein the symbols U, V, W, X, Y, Z represent carbon atoms, and pharmaceutically acceptable salts thereof.

In a thirty-third embodiment, the invention comprises the method of the thirty-second embodiment, wherein the ring defined by U-V-W-X-Y-Z is selected from the group consisting of cyclohexane, cyclohex-2-ene, cyclohex-3-ene, cyclohex-1,4-diene, cyclohex-1,5-diene, cyclohex-2,4-diene, and cyclohex-2,5-diene.

In a thirty-fourth embodiment, the invention comprises a composition comprising a functional NMDA receptor antagonist and an SSRI that is citalopram or escitalopram, and pharmaceutically acceptable salts thereof, and a pharmaceutically acceptable carrier.

In a thirty-fifth embodiment, the invention comprises the composition of the thirty-fourth embodiment, wherein the NMDA receptor antagonist is memantine hydrochloride and the SSRI is citalopram hydrobromide.

In a thirty-sixth embodiment, the invention comprises the composition of the thirty-fourth embodiment, wherein the NMDA receptor antagonist is memantine hydrochloride and the SSRI is escitalopram oxalate.

In a thirty-seventh embodiment, the invention comprises the composition of the thirty-fourth embodiment, wherein the NMDA receptor antagonist is neramexane mesylate and the SSRI is citalopram hydrobromide.

In a thirty-eighth embodiment, the invention comprises the composition of the thirty-fourth embodiment, wherein the NMDA receptor antagonist is neramexane mesylate and the SSRI is escitalopram oxalate.

In a thirty-ninth embodiment, the invention comprises the composition of the thirty-fourth embodiment, which is a solid oral dosage form.

## Claims

1. An N-methyl-D-aspartate (NMDA) receptor antagonist, or a pharmaceutically acceptable salt thereof, in combination with a compound selected from the group consisting of a selective serotonin reuptake inhibitor (SSRI), a norepinephrine reuptake inhibitor (NARI), a dual selective serotonin reuptake inhibitor/norepinephrine reuptake inhibitor (SNRI) and a tricyclic antidepressant (TCA), for use in the treatment of a mood disorder.

2. The combination as claimed in claim 1, wherein the NMDA receptor antagonist is an 1-aminocyclohexane derivative.

3. The combination as claimed in either of claims 1 or 2, wherein the NMDA receptor antagonist is represented by the general formula (I): 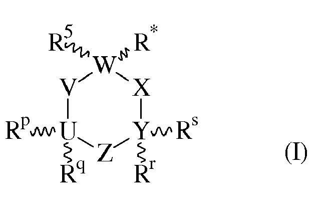 wherein:
R^{*} is -(A)ₙ-(CR¹R²)ₘ-NR³R⁴, n+m = 0,1 or 2,
A is selected from linear or branched C₁₋₆ alkylene, linear or branched C₂₋₆ alkenylene and linear or branched C₂₋₆ alkynylene,
R¹ and R² are independently selected from hydrogen, linear or branched C₁-₆ alkyl, linear or branched C₂₋₆ alkenyl, linear or branched C₂₋₆ alkynyl, aryl, substituted aryl and arylalkyl,
R³ and R⁴ are independently selected from hydrogen, linear or branched C₁₋₆ alkyl, linear or branched C₂₋₆ alkenyl and linear or branched C₂₋₆ alkynyl, or together form C₂₋₁₀ alkylene or C₂₋₁₀ alkenylene or together with the N form an optionally C₁₋₆ alkyl- or C₂₋₆ alkenyl-substituted 3-7-membered azacycloalkane or azacycloalkene; or independently R³ or R⁴ may join with R^{p}, R^{q}, R^{r} or R^{s} to form an alkylene chain -CH(R⁶)-(CH₂)ₜ,
wherein t = 0 or 1 and the left side of the alkylene chain is attached to U or Y and the right side of the alkylene chain is attached to N, and R⁶ is selected from hydrogen, linear or branched C₁₋₆ alkyl, linear or branched C₂₋₆ alkenyl, linear or branched C₂₋₆ alkynyl, aryl, substituted aryl and arylalkyl; or independently R³ or R⁴ may join with R⁵ to form an alkylene chain represented by the formula -CH₂-CH₂-CH₂-(CH₂)ₜ- or an alkenylene chain represented by the formulae -CH=CH-CH₂-(CH₂)ₜ-, -CH=C=CH-(CH₂)ₜ- or - CH₂-CH=CH-(CH₂)ₜ-, wherein t = 0 or 1 and the left side of the alkylene or alkenylene chain is attached to W and the right side of the alkylene ring is attached to N;
R⁵ is independently selected from hydrogen, linear or branched C₁₋₆ alkyl, linear or branched C₂-₆ alkenyl and linear or branched C₂₋₆ alkynyl, or R⁵ combines with the carbon to which it is attached and the next adjacent ring carbon atom to form a double bond,
R^{p}, R^{q}, R^{r} and R^{s} are independently selected from hydrogen, linear or branched C₁₋₆ alkyl, linear or branched C₂₋₆ alkenyl, linear or branched C₂₋₆ alkynyl, C₃₋₆ cycloalkyl, aryl, substituted aryl and arylalkyl, or R^{p}, R^{q}, R^{r} and R^{s} independently may form a double bond with U or with Y or to which it is attached, or R^{p}, R^{q}, R^{r} and R^{s} may combine together to represent a C₂₋₅ alkylene or alkenylene bridge which may in turn combine with R⁵ to form an additional C₁₋₃ alkylene or alkenylene bridge;
and the symbols U, V, W, X, Y and Z represent carbon atoms, including optical isomers, diastereoisomers, polymorphs, enantiomers, hydrates, pharmaceutically acceptable salts, and mixture of compounds within formula (I).

4. The combination as claimed in any of claims 1 to 3, wherein the ring defined by U-V-W-X-Y-Z in formula (I) is selected from cyclohexane, cyclohex-2-ene, cyclohex-3-ene, cyclohex-1,4-diene, cyclohex-1,5-diene, cyclohex-2,4-diene and cyclohex-2,5-diene.

5. The combination as claimed in any of claims 1 to 4, wherein the NMDA receptor antagonist is neramexane, optical isomers, diastereoisomers, polymorphs, enantiomers, hydrates and pharmaceutically acceptable salts thereof.

6. The combination as claimed in any of claims 1 to 5, wherein the SSRI is selected from the group consisting of fluoxetine, paroxetine, citalopram, sertraline, fluvoxamine , escitalopram, enantiomers and pharmaceutically acceptable salts thereof.

7. The combination as claimed in any of claims 1 to 5, wherein the SSRI is buproprion.

8. The combination as claimed in any of claims 1 to 5, wherein the NARI is selected from the group consisting of reboxetine, atomoxetine and pharmaceutically acceptable salts thereof.

9. The combination as claimed in any of claims 1 to 5, wherein the SNRI is selected from the group consisting of venlafaxine, milnacipran, duloxetine and pharmaceutically acceptable salts thereof.

10. The combination as claimed in any of claims 1 to 5, wherein the TCA is a primary, secondary, tertiary or substituted amine, preferably selected from the group consisting of amitriptyline, clomipramine, trimipramine, doxepin, nortriptyline, desipramine and protriptyline.

11. The combination as claimed in any of claims 1 to 5, wherein the TCA is selected from imipramine and pharmaceutically acceptable salts thereof, preferably imiprmaine hydrochloride.

12. The composition as claimed in any of claims 1 to 6, wherein the NMDA receptor antagonist is neramexane mesylate and the SSRI is citalopram hydrobromide or escitalopram oxalate.

13. The combination as claimed in any of claims 1 to 12, wherein the mood disorder is selected from the group consisting of depression, major depression, bipolar disorder, unipolar depression, dysthymia, cyclothemia, seasonal affective disorder, and post-partum depression.

14. The combination as claimed in claim 13, wherein said depression is secondary depression resulting from a systemic or neurological disease.

15. The combination as claimed in claim 14, wherein said systemic disease is infection, endocrine disorder, collagen vascular disease, nutritional deficiency or neoplastic disease or said neurological disease is multiple sclerosis, Parkinson's disease, Alzheimer's disease, head trauma, cerebral tumors, post-stroke, early dementia or sleep apnea.
